# EUROPEAN PATENT APPLICATION

(11) **EP 2 110 383 A1**
(43) Date of publication of application: **21.10.2009**
(21) Application number: 08450054.5
(22) Date of filing: 14.04.2008
(51) Int. Cl.: C07K 14/415, A61K 39/35

(54) **Hypoallergenic variants**

(71) Applicant: BIOMAY AG, 1090 Wien (AT)
(72) Inventor: Ferreira, Fatima, 5020 Salzburg (AT); Gadermaier, Gabriele, 5300 Hallwang (AT); Bohle, Barbara, 1040 Vienna (AT); Jahn-Schmid, Beatrice, 1090 Vienna (AT)
(74) Representative: Sonn & Partner Patentanwälte

(57) **Abstract**

The present invention relates to a hypoallergenic variant of the mugwort allergen Art v 1 (SEQ ID No. 1) or a fragment thereof, wherein the variant comprises at least one modification of at least one cysteine residue at amino acid positions 22, 26, 47 or 49 of SEQ ID No. 1.

## Description

The present invention relates to a hypoallergenic variant of the mugwort allergen Art v 1 or a fragment thereof.

Mugwort (*Artemisia vulgaris*) is a common weed plant widely spread in temperate and humid zones of Europe, along the Mediterranean basin, China, Korea, Japan, Russia, Iran, India, and North Africa. Mugwort originated from Asia, spread throughout Europe and was later introduced in the Northeastern parts of Canada and USA. In Europe, China, and Korea mugwort is the major cause of pollen allergy in late summer and affects about 10-14% of all the patients suffering from pollinosis. In addition, mugwort allergy is associated with allergic symptoms induced by ragweed pollen and certain food allergens due to IgE-cross-reactivity. Mugwort pollen contains several allergens in the range of 9 to >60 kDa including minor allergens such as Art v 3 (10 kDa; lipid transfer protein), Art v 4 (14 kDa; profilin), Art v 5 (9 kDa; Ca⁺⁺-binding protein) to which 10-40 % of mugwort allergic individuals are sensitised. The major allergen, Art v 1, however, is recognized by >95% of mugwort allergic patients. This 28 kDa glycoprotein has an unusual modular structure, being composed of a cystein-rich globular (defensin-like) domain and a hydroxy-proline-rich tail containing uncommon O-linked carbohydrate structures built from galactose and arabinose, which are partly involved in IgE-binding (Himly M,. et al FASEB J 2003; 17:106-8). The 55 amino acids long N-terminal domain of Art v 1 was compared with sequences of 14 representative members of the plant defensin family. Conserved residues in described plant defensins and the Art v 1 sequence were restricted to the eight cysteines, two glycines at position 15 and 35, an aromatic residue at position 13, and a glutamic acid at position 30 (numbering relative to Art v 1). The superfamily of defensins comprises widely distributed cysteine-rich antimicrobial peptides, differing in length, number of cysteine residues, disulfide bridges, or folding pattern. For example, insect (34-43 amino acids) and mammalian (29-34 amino acids) defensin possess three whereas plant defensins (45-55 amino acids) have four intramolecular disulfide bridges. Plant defensins are small, basic peptides with a characteristic three-dimensional folding pattern that is stabilized by eight disulfide-linked cysteines and can be found ubiquitous throughout the plant kingdom. Most plant defensins exhibit antifungal activity against a broad range of fungi, including various plant pathogens; but these proteins do normally not display antibacterial activity in contrast to defensins isolated from insects or mussels. The antimicrobial activity of plant defensins displays similar modes of action and can be classified into five groups. Groups I (e.g. pea, radish, horse chestnut defensins) and II (e.g. wheat defensin) are proteins with and without anti-fungal activity, respectively. Groups III and IV (e.g. scorpion neurotoxins) represent sodium and potassium channel blockers; whereas proteins belonging to Group V (e.g. flesh fly defensin) possess anti-bacterial activity. Plant defensins were found to be expressed predominantly in seeds and upon wounding, expression of these proteins involved in innate plant immunity was shown to be dramatically increased, thus contributing to the protection of seeds for enhanced seedling survival rates. In addition, plant defensins appear to be mainly expressed in peripheral cell layers and stomatal cells, which is consistent with a role in a first line of defense against pathogens.

Based on the sequence homology Art v 1 can be classified into the plant defensin family. Although no correlation between surface charge distribution and the presence of anti-fungal activity could be established among plant defensin, the defensin domain of Art v 1 and the anti-fungal protein Ah-AMP1 from horse chestnut (*Aesculus hippocastanum*) have similar surface charge distributions. In addition, amino acid residues Thr12, Ser 14, Phe51, Trp29, Phe48, Lys39, Arg40 His/Lys44, and Gln/Lys8 (numbering relative to Art v 1) predicted to be important for the potent anti-fungal activity and specificity of Ah-AMP1, are also conserved in the Art v 1 molecule.

It is an object of the present invention to provide variants of the allergen Art v 1 which can be employed as a vaccine for preventing or treating allergy against mugwort and cross-reacting pollen allergens.

Therefore the present invention relates to a hypoallergenic variant of the mugwort allergen Art v 1 (SEQ ID No. 1; AGSKLCEKTSKTYSGKCDNKKCDKKCIEWEKAQHGACHKREAGKESCFCYFDCSKS PPGATPAPPGAAPP-PAAGGSPSPPADGGSPPPPADGGSPPVDGGSPPPPSTH) or a fragment thereof, wherein the variant comprises at least one modification of at least one cysteine residue at amino acid positions 22, 26, 47 or 49 of SEQ ID No. 1.

Wild-type Art v 1 (SEQ ID No. 1), the major mugwort pollen allergen, is known to comprise 8 cysteine residues which are also responsible for the three-dimensional structure of the allergen. These 8 cysteine residues are located at amino acid positions 6, 17, 22, 26, 37, 47, 49 and 53 of Art v 1 (SEQ ID No. 1). Therefore, it may be assumed that the modification of one of said cysteine residues will lead to the loss of the natural three-dimensional structure of the allergen and, thus, results in a hypoallergenic molecule which shows a reduced binding to Art v 1 specific IgE antibodies. However, it surprisingly turned out that only the modification of some of said cysteine residues results in hypoallergenic molecule. These cysteine residues are those at amino acid positions 22, 26, 47 and 49 of Art v 1 (SEQ ID No. 1). If one or more of said cysteine residues are modified (e.g. by substitution) the obtained allergen variant shows hypoallergenic properties. In contrast thereto, the modification of the cysteine residue at amino acid position 6 or 53 of Art v 1 does not substantially affect the IgE reactivity.

Of course, it is also possible to additionally modify one or more of the other four cysteine residues present in the wild-type Art v 1 molecule (namely cysteine residues 6, 17, 37 and 53 of Art v 1 (SEQ ID No. 1)), provided that at least one of the cysteine residues at amino acid positions 22, 26, 47 and 49 of Art v 1 (SEQ ID No. 1) is also modified.

Possible combinations of Art v 1 modifications include cysteine residues 22, 26, 47 and/or 49 and at least one cysteine residue 6, 17, 37 and 53 of Art v 1 (SEQ ID No. 1).

The term "fragment thereof", as used herein, refers to a part of the Art v 1 molecule which harbours at least one of the T-cell epitopes of Art v 1. The T-cell epitope(s) of said fragment is (are) responsible for the induction of an immune response against said fragment as well as against the wild-type Art v 1 and other cross-reacting allergens. Furthermore the fragment of the present invention shows a significantly reduced IgE reactivity. The fragments of Art v 1 as used herein preferably comprise at least 6, preferably at least 7, more preferably at least 8, consecutive amino acid residues of SEQ ID No. 1. Furthermore the present fragments of the present invention is designed to comprise also at least one preferably at least two, three or even four of amino acid residues 22, 26, 47 and 49.

The presence of T cell epitopes in a polypeptide or peptide can be determined by methods known in the art (e.g. proliferation tests in TCL and TCC).

"Hypoallergenic", as used herein, refers to the ability of the variants of the present invention or fragments thereof to induce the production of antibodies (preferably of the IgG class) specifically binding to said allergen and exhibiting reduced or no allergic reactions when administered to an individual (reduced or no IgE reactivity compared to the wild-type allergen Art v 1). The reduced or missing ability of "hypoallergenic" variants of an allergen to induce an allergic reaction in an individual is obtained by removing or destroying the IgE binding epitopes from said allergen, however, by conserving the T cell epitopes present on said allergen. For Art v 1 this can be achieved by modifying one or more of the cysteine residues at amino acid positions 22, 26, 47 and 49 of Art v 1 (SEQ ID No. 1).

For therapeutic purposes hypoallergenic variants derived from allergens like Art v 1 should not bind IgE specific for Art v 1 or bind such IgE to a substantially lesser extent (e.g. at least 100 fold less and more preferably, at least 1000 fold less binding) than the corresponding purified native Art v 1. IgE binding activity of peptides can be determined, for example, with an enzyme linked immunosorbent assay (ELISA) using, for example, sera obtained from an individual (i.e. an allergic individual) that has been previously shown to react with native or recombinant Art v 1 in in vitro assays. Briefly, a peptide to be tested is coated onto wells of a microtiter plate. After washing and blocking the wells, antibody solution consisting of the serum or plasma of an allergic individual who has been exposed to the natural allergen is incubated in the wells. The plasma is generally depleted of IgG before incubation. However, the depletion is not necessary if highly specific anti IgE-antibodies are used. Furthermore, allergic individuals who have not undergone specific immunotherapy have in some cases almost no detectable IgG antibodies specific for the allergen in question. A labelled secondary antibody is added to the wells and incubated. The amount of IgE binding is then quantified and compared to the amount of IgE bound by a purified native Art v 1 protein. Alternatively, the binding activity of a peptide can be determined by Western blot analysis. For example, a peptide to be tested is run on a polyacrylamide gel using SDS-PAGE. The peptide is then transferred to nitrocellulose and subsequently incubated with sera from an allergic subject. After incubation with the labelled secondary antibody, the amount of IgE bound is then determined and quantified.

Another assay which can be used to determine IgE binding activity of a peptide is a competition ELISA assay. Briefly, an IgE antibody pool is generated by combining plasma or serum from mugwort pollen allergic individuals that have been shown by direct ELISA to have IgE reactive with native Art v 1. This pool is used in ELISA competition assays to compare IgE binding of native Art v 1 and the hypoallergenic variant of interest. IgE binding for the native Art v 1 protein and the variant being tested is determined and quantified.

Furthermore, the hypoallergenic variants (polypeptides or peptides) of the present invention do preferably not result in the release of mediators (e.g. histamines) from mast cells or basophils. To determine whether a peptide which binds IgE results in the release of mediators, a histamine release assay can be performed using standard reagents and protocols. Briefly, a buffered solution of a peptide to be tested is combined with an equal volume of whole heparinized blood from an allergic subject. After mixing and incubation, the cells are pelleted and the supernatants are processed and analyzed using, e.g., a radioimmunoassay to determine the amount of histamine released.

According to a preferred embodiment of the present invention the at least one modification comprises an amino acid substitution, an amino acid deletion or a chemical modification in which the sulfur group of the cysteine residue is joined to a carbon atom or sulfur atom of a sulfur blocking group. Sulfhydryl groups in proteins are highly reactive and can rapidly undergo covalent reactions with several blocking agents. Such sulfur blocking agents can be permanent, which form stable thioester linkages, or reversible, which form disulfide bonds susceptible to cleavage by addition of an appropriate reducing agent. Examples of permanent blocking agents are N-Ethylmaleimide, Iodoacetate or bromoacetate. Examples of reversible or temporary blocking agents are sodium tetrathionate, Ellman's Reagent 5,5'-dithio-bis(2-nitrobenzoic acid), 4,4'-dipyridyl disulfide and 2,2'-dipyridyl disulfide (Bioconjugate Techniques, Greg T. Her-manson, Academic Press 1996).

The cysteine residues at amino acid positions 22, 26, 47 and 49 of Art v 1 (SEQ ID No. 1) can be modified by various methods, provided that the modification results in a hypoallergenic molecule which is no longer able to form at defined positions intramolecular disulfide bridges. The inability to form such disulfide bridges leads to a variant of Art v 1 which does not or substantially not exhibit the three-dimensional structure of the wild-type Art v 1. The most efficient way to prevent the formation of disulfide bridges is the deletion of the respective cysteine residues or the substitution of the cysteine residues with amino acid residues which are not able to form such bridges. Particularly preferred amino acid residues which can be used to substitute the cysteine residues are selected from the group consisting of serine, alanine, or glycine.

Alternatively it is of course possible to modify the cysteine residues at amino acid positions 22, 26, 47 and 49 of Art v 1 (SEQ ID No. 1) so that the free sulfur group of at least one of said cysteine residues is blocked by a blocking group. Examples of permanent blocking agents are N-Ethylmaleimide, Iodoacetate or bromoacetate. Examples of reversible or temporary blocking agents are sodium tetrathionate, Ellman's Reagent 5,5'-dithio-bis(2-nitrobenzoic acid), 4,4'-dipyridyl disulfide and 2,2'-dipyridyl disulfide (Bioconjugate Techniques, Greg T. Her-manson, Academic Press 1996).

According to a preferred embodiment of the present invention the fragment comprises amino acid residues 1 to 55 of SEQ ID No. 1, preferably amino acid residues 19 to 36 of SEQ ID No. 1, more preferably amino acid residues 23 to 36 of SEQ ID No. 1, in particular amino acid residues 25 to 36 of SEQ ID No. 1.

From previous studies it is known that the T-cell epitopes of Art v 1 are located at the N-terminus of Art v 1 ("defensin-like domain"). Therefore it is preferred that the fragment according to the present invention comprises/consists of amino acid residues 1 to 55 of SEQ ID No. 1, preferably amino acid residues 19 to 36 of SEQ ID No. 1, more preferably amino acid residues 23 to 36 of SEQ ID No. 1, in particular amino acid residues 25 to 36 of SEQ ID No. 1. The utilization of N-terminal fragments consisting of the defensin-like domain without the proline-rich part assay be advantageous in terms of recombinant production. Production of the defensin domain alone compared to the full-length protein may also be an alternative, as the yield of recombinant protein is 2-3 folds higher and degradation products are not abundant. Thus, from the production point of view, the defensin domain has advantages than Art v 1 full-length protein and mutants thereof. As the immunological properties of the proline domain produced in bacteria may be irrelevant, and the defensin domain is immunologically comparable to the full-length protein, the production of this domain or mutants thereof are preferred.

According to a preferred embodiment of the present invention the variant comprises/consists of an amino acid sequence selected from the group consisting of SEQ ID No. 2 to 13.

Another aspect of the present invention relates to a nucleic acid molecule encoding a hypoallergenic variant according to the present invention.

The nucleic acid molecule of the present invention may be employed, e.g., for the recombinant production of the peptides/polypeptides/proteins encoded by said nucleic acid molecule. Furthermore, they may also be used for therapeutic aspects (e.g. gene therapy, cell therapy).

Yet another aspect of the present invention relates to a vector comprising a nucleic acid molecule according to the present invention.

Preferred vectors (e.g. plasmids) to be used according to the present invention are cloning as well as expression vectors comprising promoters, an origin of replication, regulatory elements, selection markers and/or other vector elements. If the vector is an integration vector able to be integrated into the genome of the host cell, corresponding means may be provided on said vector (e.g. insertion sequence elements). The type of vector used and the regulatory elements present on said vector also depend on the host cell into which said vector will be integrated. If expression vectors are used, the DNA molecule according to the present invention and encoding a polypeptide as described above, is operably linked to a promoter region.

Preferably, the vector of the present invention further comprises a promoter operably linked to said nucleic acid molecule, thus resulting in an expression cassette.

The expression cassette of the present invention comprises a promoter and a nucleic acid molecule encoding for a peptide of the present invention. The promoter is preferably positioned at the 5'-end (upstream) of the nucleic acid molecule of the present invention. The promoter to be used in the expression cassette may be any one, provided that the promoter can be controlled by the respective host.

Another aspect of the present invention relates to a host cell comprising a nucleic acid molecule or a vector according to the present invention.

The host cell according to the present invention can be used to recombinantly produce the hypoallergenic variant of the present invention. The cell to be used herein may be a eukaryotic as well as a prokaryotic cell. Preferred eukaryotic cells are yeast cells, in particular *Saccharomyces cerevisiae, Pichia pas-toris* and *Hansenula polymorpha*, plant cells, in particular tobacco plant cells, insect cells and mammalian cells, like human and animal cells, in particular Chinese hamster ovary cells. Preferred prokaryotic cells are, e.g., *Bacillus subtilis* and *Es-cherichia coli*. The vector or nucleic acid molecule encoding the hypoallergenic variant of the present invention is chosen based on the host cell used. Means and methods for producing host cells according to the present invention are well known to the person skilled in the art.

A further aspect of the present invention relates to a pharmaceutical preparation comprising at least one hypoallergenic variant, a nucleic acid molecule or a vector according to the present invention.

The hypoallergenic variant of the present invention or nucleic acid molecule encoding said variant can be used in a vaccine formulation. Since these molecules are able to provoke a T cell response against the mugwort pollen allergen Art v 1 the pharmaceutical formulation may be used for the desensitization, prevention or treatment of mugwort pollen allergies and allergies cross-reacting with mugwort pollen allergens (e.g. similar allergens present in botanically-related and un-related plant species. Due to the reduced IgE binding reactivity the variants are particularly advantageous when used in a vaccine, because no or substantially no allergic reaction is provoked by the application of such a vaccine. The vaccine formulation of the present invention may comprise at least one, preferably at least two, more preferably at least three, peptides of the present invention.

Administration of the therapeutic compositions of the present invention to an individual to be desensitized can be carried out using known procedures at dosages and for periods of time effective to reduce sensitivity (i.e. to reduce the allergic response) of the individual to the allergen. Effective amounts of the therapeutic compositions will vary according to factors such as the degree of sensitivity of the individual to Art v 1, the age, sex and weight of the individual and the ability of the protein or fragment thereof to elicit an antigenic response in the individual. The active compound (i.e. protein or fragment thereof) may be administered in a convenient manner such as by injection (subcutaneous, intravenous, etc.), oral administration, inhalation, transdermal application, or rectal administration. Depending on the route of administration, the active compound may be coated within a material to protect the compound from the action of enzymes, acids and other natural conditions which may inactivate the compound. For example, preferably about 0.05 µg-1000 µg, more preferably from about 0.1-100 µg of active compound (i.e. protein or fragment thereof) per dosage unit may be administered by injection. Dosage regimen may be adjusted to provide the optimum therapeutic response. For example, several divided doses may be administered daily or the dose may be proportionally reduced as indicated by the exigencies of the therapeutic situation.

The pharmaceutical/vaccine formulation of the present invention further comprises at least one pharmaceutical acceptable adjuvant, excipient and/or carrier.

Pharmaceutically acceptable carriers preferably used are physiological saline, vegetable oils, mineral oil, aqueous sodium caroboxymethyl cellulose or aqueous polyvinylpyrrolidone. Suitable adjuvants include, but are not limited to: surface active substances, e.g., hexadecylamine, octadecylamine, octadecyl amino acid esters, lysolecithin, dimethyl-dioctadecylammonium bromide, methoxyhexadecylgylcerol, and pluronic polyols; polyamines, e.g., pyran, dextran-sulfate, poly IC, carbopol; peptides, e.g., muramyl dipeptide, dimethylglycine, tuftsin; oil emulsions; and mineral gels, e.g., aluminum hydroxide, aluminum phosphate, etc. and immune stimulating complexes. The adjuvant may be, for example, alum or a composition containing a vegetable oil, isomannide monooleate and aluminum mono-stearate. Other preferred adjuvants include microparticles or beads of biocompatible matrix materials. The molecules of the present invention may be incorporated into microparticles or microcapsules to prolong the exposure of the antigenic material to the individual and hence protect said individual against infection for long periods of time. The immunogen may also be incorporated into liposomes or conjugated to polysaccharides and/or other polymers for use in a vaccine formulation.

Also part of this invention is a composition that comprises the molecules, in particular the peptides, of this invention and a carrier, preferably a biologically-acceptable carrier, and more preferably a pharmaceutically-acceptable carrier. Typical carriers are aqueous carriers such as water, buffered aqueous solutions, aqueous alcoholic mixtures, and the like. Compositions comprising carriers that are for pharmaceutical use, particularly for use in humans, comprise a carrier that is pharmaceutically-acceptable. Examples of such carriers are known in the art.

Typically, such vaccines are prepared as injectables: either as liquid solutions or suspensions, solid forms suitable for solution in, or suspension in, liquid prior to injection may also be prepared. The vaccine may be administered to a target animal or an allergic patient by any convenient route, such as subcutaneously, intraperitoneally, intramuscularly, intradermally, intravenously, orally, intranasally or intramammar-ily, in the presence of a physiologically acceptable diluent. The antigens may be administered in a single dose or in a plurality of doses. The vaccine of the present invention may be stored under refrigeration or in frozen or lyophilized form. The vaccine is administered to an individual in an amount effective to elicit a protective immune response as compared to a control. The effective amount will vary, e.g., with the age and size and may be readily determined by the practitioner skilled in the art. Suitable regimes for initial administration and booster shots will also be variable, but may be typified by an initial administration followed by subsequent inoculations or other administrations.

The hypoallergenic variants of the present invention may be combined with other hypoallergenic variants known in the art, so that by the application of one single pharmaceutical/vaccine formulation not only mugwort allergy may be treated and/or prevented but also other allergies (see e.g. WO 2006/058359, WO 2007/124524, WO 2007/124526 etc. Of course the hypoallergenic variants according to the present invention may be administered as a single vaccine or in combination. It is also possible to fuse the variants of the present invention to another molecule.

Another aspect of the present invention relates to the use of at least one hypoallergenic variant, a nucleic acid molecule or a vector according to the present invention for the manufacture of a pharmaceutical preparation, preferably vaccine, for preventing or treating a mugwort pollen allergy in an individual, in particular an allergy caused by Art v 1, or an allergy cross-reacting with a mugwort pollen allergy.

The present invention is further illustrated by the following figures and examples, however, without being restricted thereto.
Fig. 1 shows serine (A) and alanine (B) variants of Art v 1 obtained by site-directed mutagenesis. Sequence alignment of eight serine and six alanine mutants of Art v 1; exchanges of cysteine residues for serine (S) are highlighted in bold, exchanges for alanine (A) are highlighted in bold.
Figure 2 shows a schematic overview of pHIS-parallel2 multiple cloning site, including hexa-histidine tag, recognition sequence for cleavage with rTEV protease, and used restriction enzymes.
Figure 3 shows IgE immunoblots of recombinant Art v 1, serine (A) and alanine (B) variants. Purified rArt v 1 and mutated proteins were subjected to SDS-PAGE and immunoblots with sera from Art v 1-allergic patients were used to demonstrate reactivity to recombinant wild type (wt) Art v 1 and fourteen cysteine variants (4 selected patients shown). Numbers on top of the panel indicate the position of amino acid exchange from cysteine to serine (A) and alanine (B).
Figure 4 shows IgE reactivity of wild-type (wt) Art v 1, serine (A) and alanine (B) mutants. Relative IgE reactivity of data obtained by immunoblot experiments with sera from mugwort pollen-allergic patients (n=12) were used to determine the mean reactivity of recombinant wt Art v 1 and variants. A virtual scale from 0-5 was applied for calculation, with 5 representing the strongest IgE reactivity and 0 for no detectable reactivity in immunoblot experiment. Numbers on bottom of the panel indicate the position of amino acid exchange from cysteine to serine (A) and alanine (B).
Figure 5 shows a Coomassie (A) and silver stained (B) SDS-PAGE of purified nArt v 1, rArt v 1 and cysteine variants C22S, C47S, and C49S. Aliquots of 4 µg for Coomassie staining and 1 µg for silver staining of the purified proteins were loaded on a 15% gel.
Figure 6 shows far UV-CD spectra of nArt v 1, rArt v 1, variants C22S, C47S, and C49S recorded at 20°C. Data is baseline subtracted, normalized and presented as mean residue molar ellipticity [Θ]_{MRW}.
Figure 7 shows IgE immunoblot of natural and recombinant Art v 1 and three variants using sera from mugwort pollen-allergic patients (1-6). Serum from a non-atopic healthy donor was used as control (NHS) and a buffer control (BC) was performed to show the specificity of the detection antibody.
Figure 8 shows ELISA of natural, recombinant and three cysteine variants of Art v 1 tested with serum IgE of 16 Art v 1-allergic patients. Variants C22S, C47S, and C49S of recombinant Art v 1 showed dramatically reduced IgE binding compared to wild type rArt v 1 (P < 0.001) and nArt v 1 (P < 0.001).
Figure 9 shows Natural Art v 1 (A) and recombinant Art v 1 (B) were immobilized on ELISA plates and cross-inhibition was performed with sera from 5 Art v 1-allergic patients. Increasing concentrations of inhibitory proteins were used to pre-incubate the patients' sera.
Figure 10 shows a box plot analysis of cross-inhibition experiments. Absolute protein amounts to obtain 50% inhibition of natural Art v 1 (A) and recombinant Art v 1 (B) were determined for each patient (n=5).

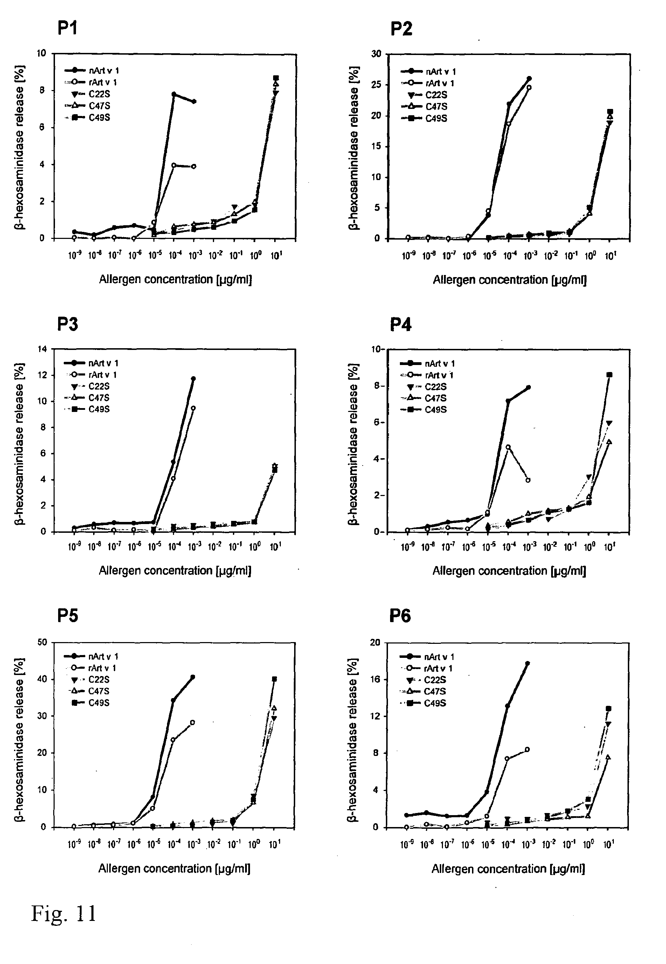

Figure 11 shows the biologic activity of nArt v 1, rArt v 1 and 3 hypoallergenic variants was determined by degranulation of transfected RBL cells. Rat basophil leukemia cells were sensitized with patients' serum IgE derived from 6 mugwort pollen-allergic individuals (P1 - P6) reacting with natural and recombinant Art v 1. Allergen-dependent mediator release was triggered by serial dilutions of nArt v 1 and rArt v 1 (10⁻⁹ - 10⁻³ µg/ml) and cysteine mutants of rArt v 1 (10⁻⁵ - 10¹ µg/ml). Degranulation was measured by β-hexosaminidase release in the supernatant and is shown as percentage of degranulation achieved for total release of mediator through cell lysis.
Figure 12 shows ELISA of full-length Art v 1 and defensin domain was performed and serum IgE binding of 18 Art v 1-allergic patients was assessed.

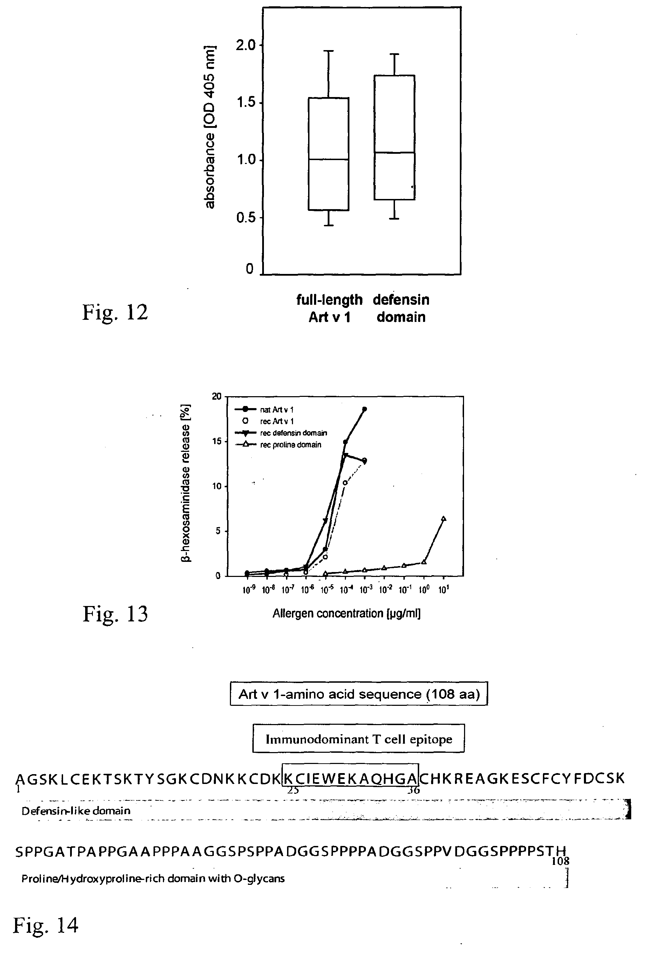

Figure 13 shows biologic activity of natural Art v 1, recombinant Art v 1, defensin and proline domain was determined by degranulation of transfected RBL cells. Rat basophil leukemia cells were sensitized with patients' serum IgE derived from 6 Art v 1- allergic patients (P1 - P6). Allergen dependent mediator release was triggered by serial dilutions of nArt v 1, rArt v 1, and defensin domain (10⁻⁹ - 10⁻³ µg/ml) and proline domain (10⁻⁵ - 10¹ µg/ml). Degranulation was measured by β-hexosaminidase release in the supernatant and is shown as percentage of degranulation achieved for total release of mediator through cell lysis.

Figure 14 shows the amino acid sequence of Art v 1.
Figure 15 shows maximum proliferation induced by Art v 1 and different Art v 1-variants in PBMC derived from mugwort pollen-allergic individuals (n=14).
Figure 16 shows the proliferation of PBMC derived from mugwort pollen-allergic individuals (n=8) induced by Art v 1 and different Defensin-variants at 5 µg/ml.
Figure 17 shows the proliferation of Art v 1-specific TCL derived from 23 different mugwort pollen-allergic patients stimulated with 5 µg/ml Art v 1, Art v 1-mutants C22S, C47S, and C49S (22 /28) or an equimolar mixture of C22S, C47S, and C49S (n=8 /28) or Defensin-variants (n=17 /28).
Figure 18 shows allergen-specific proliferation of representative TCL or TCC derived from mugwort-allergic individuals and stimulated with different concentrations of Art v 1- (A and B) or Defensin-variants (C).
Figure 19 shows the comparison of concentration-dependent proliferation in response to rArt v 1 and Defensin-domain for one representative Art v 1-specific TCL.
Figure 20 shows the comparison of proliferation induced by rArt v 1, Art v 1-mutants and defensin-variants in one representative Art v 1-specific TCL. Amino acids relevant for the T cell response are highlighted in bold.
Figure 21 shows the comparison of proliferation induced by Art v 1, Art v 1-mutants and defensin-variants in 2 representative Art v 1-specific TCC. Amino acids relevant for T cell response are highlighted in bold.
Figure 22 shows an inhibition-ELISA. Preincubation with nArt v 1 and rArt v 1 inhibits binding of human IgE serum-pool (n=10 mugwort allergic patients) to Art v 1. No significant inhibition of binding was observed after pre-incubation of the serum with Art v 1-peptides or with the negative controls bovine serum albumin or an irrelevant 12-mer peptide from Bet v 1.
Figure 23 shows skin prick tests with 3 mugwort pollen allergic patients (A, B, C). Mugwort extract, rArt v 1 and peptides (12-mer, 14-mer, and 18-mer) were tested for *in vivo* IgE reactivity at concentrations up to 100 µg/ml
Figure 24 shows MdDC from two individuals were incubated for 48 hours with medium alone, LPS (100 ng/ml), rBet v 1, rArt v 1, 12-, 14- or 18-mer peptides (10µg/ ml each) and surface marker expression was measured by flow cytometry.
Figure 25 shows the proliferation of PBMC in response to Art v 1-peptides.
Figure 26 shows proliferation of Art v 1-specific T cell lines in response to stimulation with Art v 1-peptides at a concentration of 5 µg/ml (A.) or 50 µg/ml (B). No. of TCL tested: 12-mer, 14-mer, 18-mer: n=13 ; AC, SC: n=12; SS, AA, SA, AS: n=9.
Figure 27 shows proliferation of Art v 1-specific TCL induced by Art v 1-peptides. Relevant core amino acids within the aa sequence of Art v 125-36 are indicated in bold.
Figure 28 shows the proliferation of 2 Art v 1-specific TCC induced by Art v 1-derived peptides. Relevant core amino acids within the aa sequence of Art v 125-36 are indicated in bold.
Figure 29 shows stimulation of Art v 1-specific TCC with Art v 1-peptides after 3 freeze-thaw-heating (FTH) cycles (1h at 37°C) or untreated control peptides. Proliferation in response to 5 µg/ml (black bars) and 50 µg/ml (white bars) is shown.

### EXAMPLES:

### Example 1: Hypoallergenic cysteine variants of Art v 1 and Art v 1-defensin domain

### 1.1.Site-directed mutagenesis of Art v 1

### Methods

Site-directed mutagenesis was performed using the QuikChange Site-Directed Mutagenesis protocol (Stratagene, CA, USA) to exchange the eight cysteine residues located in the Art v 1 protein. The cDNA sequence coding for the mature Art v 1 protein was previously cloned into the expression plasmid pMW 172b (Himly et al., FASEB, (17) (2003):106-108). For generation of the Art v 1-cysteine variants, isoform "a" was used as template for all mutagenesis experiments. Cysteine residues located in the sequence were replaced in turn for serine and alanine, which represent the most common amino acids for "safe" substitution of cysteine residues. The bases TGT, TGC coding for cysteine were replaced by TCT, TCC for serine exchange, and GCT, GCC for alanine exchange, respectively. Corresponding reverse primers containing the mismatch were designed to anneal to the same sequence on opposite side of the template plasmid DNA. Mutagenic primers were extended using PfuTurbo DNA polymerase and the parental, non-mutated DNA template was digested with Dpn I endonuclease for 2 hours at 37°C.

Transformation of mutagenized DNA into E.coli XL1-Blue MRF' was performed using a MicroPulser and colonies were selected on LB plates containing 100 mg/l ampicillin. Obtained plasmids were sequenced according to the Dye Terminator Cycle Sequencing protocol (Applied Biosystems) to confirm the introduced base pair exchanges.

**Table 1. Forward oligonucleotides for generation of serine and alanine variants**

| | | SEQ ID No. |
|---|---|---|
| C6S-fwd | 5'-GCTGGTTCAAAGTTG**TCT**GAAAAGACAAGCAAGACG-3' | 14 |
| C6A-fwd | 5'-GCTGGTTCAAAGTTG**GCT**GAAAAGACAAGCAAGACG-3' | 15 |
| C17S-fwd | 5'-GACGTATTCGGGTAAG**TCC**GACAACAAGAAATGTGAC-3' | 16 |
| C17A-fwd | 5'-GACGTATTCGGGTAAG**GCC**GACAACAAGAAATGTGAC-3' | 17 |
| C22S-fwd | 5′-CGGGTAAGTGCGACAACAAGAAATCTGACAAAAAGTGTATA- GAGTGGG-3′ | 18 |
| C22A-fwd | 5′-CGGGTAAGTGCGACAACAAGAAAGCTGACAAAAAGTGTATA- GAGTGGG-3′ | 19 |
| C26S-fwd | 5'-GTGACAAAAAG**TCT**ATAGAGTGGGAGAAAGCGC-3' | 20 |
| C26A-fwd | 5'-GTGACAAAAAG**GCT**ATAGAGTGGGAGAAAGCGC-3' | 21 |
| C37S-fwd | 5'-CAACATGGTGCT**TCT**CACAAGAGAGAAGCCGGC-3' | 22 |
| C47S-fwd | 5'-GCCGGCAAAGAAAGT**TCC**TTTTGCTACTTTGACTGTCC-3' | 23 |
| C49S-fwd | 5'-GCCGGCAAAGAAAGTTGCTTT**TCC**TACTTTGACTGTTCC-3' | 24 |
| C49A-fwd | 5'-GCCGGCAAAGAAAGTTGCTTT**GCC**TACTTTGACTGTTCC-3' | 25 |
| C53S-fwd | 5'-GCTTTTGCTACTTTGAC**TCT**TCCAAATCGCCTCCTGG-3' | 26 |
| C53A-fwd | 5'-GCTTTTGCTACTTTGAC**GCT**TCCAAATCGCCTCCTGG-3' | 27 |

**Table 2. Reverse complementary oligonucleotides for generation of serine and alanine variants**

| | | SEQ ID No. |
|---|---|---|
| C6S-rev | 5'-CGTCTTGCTTGTCTTTTC**AGA**CAACTTTGAACCAGC-3' | 28 |
| C6A-rev | 5'-CGTCTTGCTTGTCTTTTC**AGC**CAACTTTGAACCAGC-3' | 29 |
| C17S-rev | 5'-CACATTTCTTGTTGTC**GGA**CTTACCCGAATACGTC-3' | 30 |
| C17A-rev | 5'-CACATTTCTTGTTGTC**GGC**CTTACCCGAATACGTC-3' | 31 |
| C22S-rev | 5′-CCCACTCTATACACTTTTTGTCAGATTTCTTGT- TGTCGCACTTACCC-3′ | 32 |
| C22A-rev | 5′-CCCACTCTATACACTTTTTGTCAGCTTTCTTGT- TGTCGCACTTACCC -3′ | 33 |
| C26S-rev | 5'-GCGCTTTCTCCCACTCTAT**AGA**CTTTTTGTCACATTTC-3' | 34 |
| C26A-rev | 5'-GCGCTTTCTCCCACTCTAT**AGC**CTTTTTGTCACATTTC-3' | 35 |
| C37S-rev | 5'-GCCGGCTTCTCTCTTGTG**AGA**AGCACCATGTTGCGC-3' | 36 |
| C47S-rev | 5'-GGAACAGTCAAAGTAGCAAAA**GGA**ACTTTCTTTGCCGGC-3' | 37 |
| C49S-rev | 5′-GGCGATTTGGAACAGTCAAAGTAG- GAAAAGCAACTTTCTTTGCC-3′ | 38 |
| C49A-rev | 5′-GGCGATTTGGAACAGTCAAAGTAG- GCAAAGCAACTTTCTTTGCC-3′ | 39 |
| C53S-rev | 5'-GGAGGCGATTTGGA**AGA**GTCAAAGTAGCAAAAGCAAC-3' | 40 |
| C53A-rev | 5'-GGAGGCGATTTGGA**AGC**GTCAAAGTAGCAAAAGCAAC-3' | 41 |

### Results

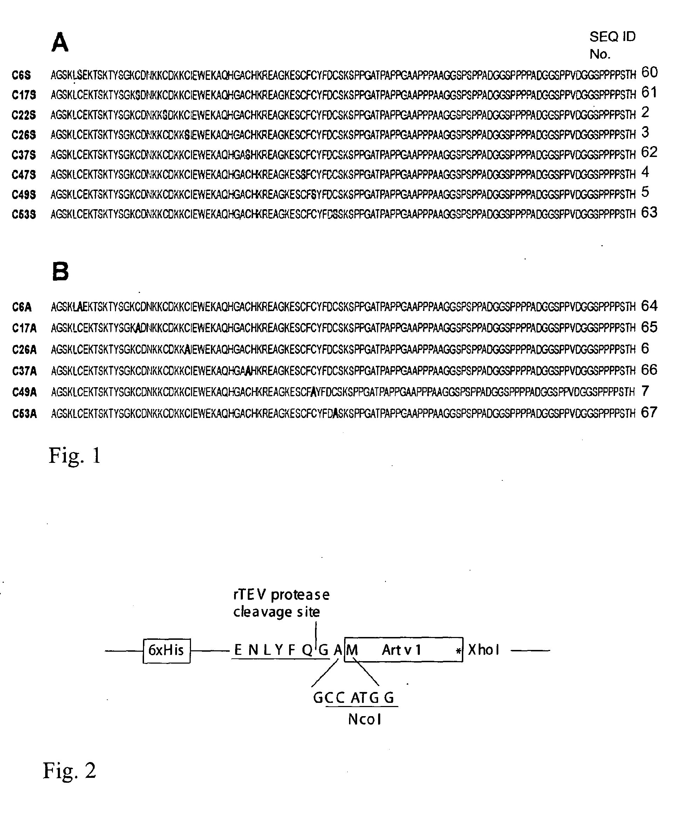

Using site directed mutagenesis a panel of eight serine and six alanine variants replacing all cysteine residues in the mature sequence of Art v 1a were obtained. Art v 1 serine variants C6S, C17S, C22S, C26S, C37S, C47S, C49S, and C53S which represent the whole panel of possible single amino acid exchanges of cysteine residues were generated. Variants C6A, C17A, C26A, C37A, C49A, and C53A were designed by exploitation of the same approach (Fig. 1).

### 1.2. Cloning of Art v 1 and variants into pHIS-parallel2

To overcome the expression problems (diminished growth of bacteria and extremely low amount of Art v 1), which were faced using the pMW172b expression vector, another system was exploited allowing the fusion of a histidine tag to the protein. The pHis-parallel2 vector system (Sheffield et al., Protein Expr Purif. 1999 Feb;15(1):34-9) was used to fuse a hexa-histidine tag N-terminal to the protein, thus facilitating the purification of even low amounts of expressed protein.

### Methods

PCR amplification of Art v 1a, eight serine and six alanine mutant constructs was conducted with primers incorporating a 5'NcoI and 3'XhoI restriction site. The following primers were used for cloning: Art v 1 fwd NcoI: 5'-GAGAGACCATGGCTGGTTCAAAGTTG-3' (SEQ ID No. 42) and Art v 1 rev XhoI: 5'-GAGAGAGACTCGAGT-TAGTGAGTGGACGGAGG- 3' (SEQ ID No. 43). Obtained DNA fragments were ligated into the respective sites of pHIS-parallel2 vector using standard molecular biology techniques. All constructs were sequenced according to the Dye Terminator Cycle Sequencing protocol.

### Results

Wild-type Art v 1, eight serine variants (C6S, C17S, C22S, C26S, C37S, C47S, C49S, and C53S) and six alanine variants (C6A, C17A, C26A, C37A, C49A, and C53A) were cloned into the pHIS-parallel2 vector system (Fig. 2).

### 1.3. Expression and purification of histidine tagged Art v 1, eight serine and six alanine variants

### Methods

For optimization of protein expression, different strains of *E. coli* i.e. BL21(DE3), BL21(DE3)pLysS, and CodonPlus BL21(DE3) strains RIL or RP cells were transformed with the plasmid DNA coding for wild-type Art v 1a. All mutant constructs were transformed into *E. coli* BL21(DE3) CodonPlus BL21(DE3) strains RIL. Several clones were used to inoculate liquid LB medium with 100 mg/l ampicillin and grown at 37°C overnight. The pre-culture was diluted 1:5 in pre-warmed media and cells were grown at 37°C to an OD₆₀₀ of 0.8. IPTG was added to a final concentration of 1 mM, and incubation continued for 4 hours at 37°C. Bacterial cells were harvested by centrifugation at 4,500 g for 20 min at 4°C and stored at -20°C.

After thawing, cells were resuspended in 1/10 culture volume 50 mM sodium phosphate pH 7.4, 300 mM NaCl, 10 mM imidazole (start buffer). Cell disruption was achieved by three cycles of freezing the cell suspension in liquid nitrogen followed by thawing at 37°C. The soluble fraction was recovered after centrifugation at 10,000 g for 30 min at 4°C. Recombinant proteins were purified by immobilized metal ion affinity chromatography (IMAC). Fast Flow Chelating Sepharose was charged with 0.1 M NiSO₄ and equilibrated with start buffer. Unspecific bound proteins were washed and histidine-tagged proteins were eluted with 50 mM sodium phosphate pH 7.4, 300 mM NaCl, containing increasing amounts of imidazole (75 mM, 150 mM, and 250 mM, respectively). Fractions were analyzed by SDS-PAGE and those containing recombinant 6 x histidine Art v 1 and Art v 1 variants in apparent purity (>90%) were dialyzed against 10 mM sodium phosphate buffer pH 7.4 and stored at -20°C.

Sera or plasma from atopic and non-atopic individuals were diluted 1:10 50 mM sodium phosphate buffer pH 7.4, 0.5 % (v/v) Tween-20, 5 % (w/v) bovine serum albumin, 0.05 % (w/v) sodium azide. Immunoblots were incubated with diluted patient's sera over night at 4°C. Bound IgE antibodies were detected with ¹²⁵I-rabbit anti-human IgE on a phosphorimager screen using a Fujifilm BAS-1800 II reader.

### Results

Different bacterial strains were tested with the Art v 1/pHIS-parallel2 construct and significant differences in the production of recombinant protein were obtained. Use of CodonPlus BL21(DE3) strains RIL and RP resulted in expression levels about 5-10 times higher than expression in BL21(DE3) and BL21(DE3)pLysS.

Art v 1 and cysteine variants were therefore expressed as fusion proteins in *E*. *coli* BL21(DE3)CodonPlus RIL. Soluble bacterial extracts were incubated with a Fast Flow sepharose complexed with Ni²⁺ and histidine tagged proteins were eluted in several steps with increasing amounts of imidazole in the elution buffer. Using non-reducing conditions, the histidine tag seemed to be partially buried in the cysteine containing domain of the protein, and thus did not result in a distinct elution peak of Art v 1. Fractions containing mainly Art v 1 proteins were pooled and protein purity was demonstrated to be about 80-90% in SDS-PAGE and Coomassie staining. Protein amounts corresponding to Art v 1 or mutant proteins were equalized using densitometry in a Coomassie stained gel and a mouse anti penta-his IgG1 antibody.

For these experiments, all proteins were produced as fusion proteins and the 6x-histidine tag was not cleaved thereafter. Thus, the cysteine variants were compared with wild type Art v 1, also expressed as fusion with a 6x-histidine tag.

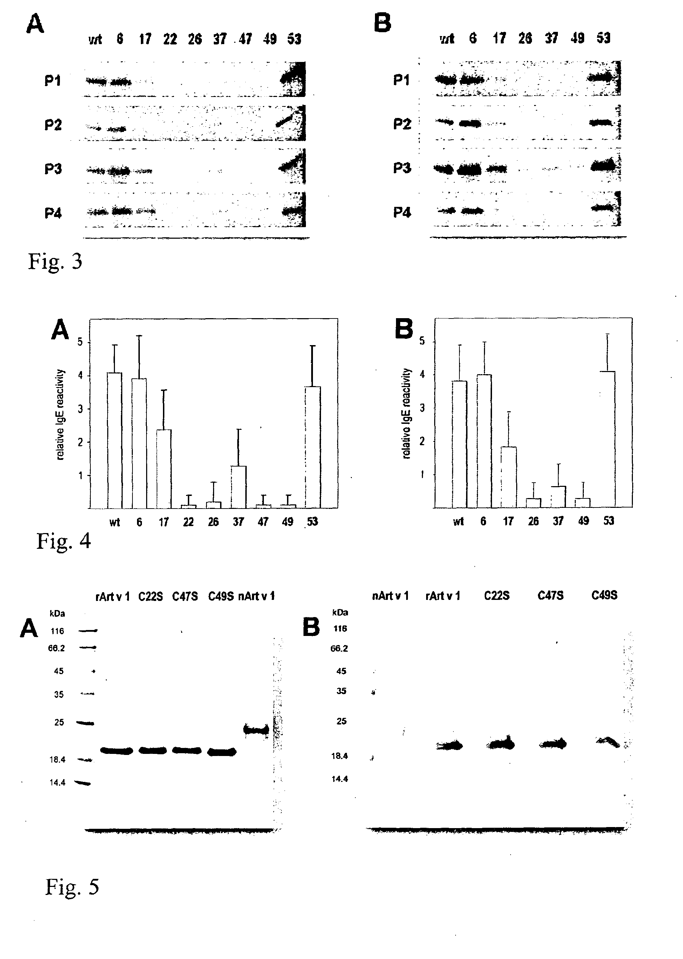

To determine the capacity of the mutated proteins to react with serum IgE of Art v 1-allergic patients immunoblot experiments were performed. Wild-type Art v 1 and all mutants were tested with serum from 12 different mugwort pollen-allergic patients, previously shown to react with natural and recombinant Art v 1 in IgE immunoblots. Cysteine mutations at position 6 and 53 showed no influence on IgE binding activity compared to wild-type Art v 1, whereas amino acid exchanges at position 17 and 37 did result in up to 50 % reduction in the ability to react with patients' IgE. Substitution of cysteine residues at positions 22, 26, 47 and 49 resulted in very low or no IgE reactivity with serum from Art v 1-allergic patients (Fig. 3 and 4). Consistent findings in the capability to bind IgE were observed for serine and alanine mutants targeting the same amino acid positions. Thus, it can be concluded that the difference in the IgE reactivity for each individual mutated protein does not result from a contribution of the introduced amino acid serine or alanine, respectively. Comparable IgE reactivity patterns were observed for pairs of cysteine residues thought to be involved in disulfide bond formation, e.g. position 6 and 53; position 17 and 37.

### 1.4. Lab-scale expression, purification and characterization of wild-type Art v 1 and three selected variants (C22S, C47S, and C49S)

Based on the results of the recombinant 6x-histidine tagged serine and alanine mutants of Art v 1 in immunoblot experiments, distinct proteins were chosen for lab-scale production and extensively characterized on biochemical and immunological basis. As lowest IgE reactivity was observed for amino acid exchanges at position 22, 26, 47 and 49 these proteins seemed to be the ideal candidates for production of hypoallergenic variants of recombinant Art v 1. A single immunodominant T cell epitope of Art v 1 was previously determined to be located in the range of amino acid 22-36 (Jahn-Schmid et al., J Immunol, 169: 6005-11, 2002).

### Methods

### Protein expression and purification

Plasmid DNA encoding Art v 1, C22S, C47S, and C49S were transformed into E. coli BL21-CodonPlus(DE3)-RIL cells and clones were selected on LB plates containing 100 mg/l ampicillin. Several clones were used to inoculate Terrific Broth (TB) medium supplemented with 50 mg/l ampicillin and grown at 30°C overnight. The pre-culture was diluted 20-fold in 5 l of the same medium and grown to an OD600 of 1.6-1.8. To induce an osmotic stress, the bacterial culture was supplemented with 0.5 M sorbitol, 4% NaCl, and 10 mM glycine betaine and incubated at 16°C for additional 30 min. Thereafter, recombinant Art v 1 expression was induced by the addition of 0.4 mM IPTG and incubation at 16°C overnight. Cells were harvested by centrifugation at 5,000 g for 30 min at 4°C and stored at -20°C.

Purification of histidine proteins was performed using the ÄKTAprime chromatographic system. Cells were harvested in 1/20 culture volume 50 mM sodium phosphate pH 7.4, 500 mM NaCl, 10 mM imidazole, 5% (v/v) glycerol. Lysozyme was added to a final concentration of 0.25 mg/ml and the suspension was incubated for 30 min at room temperature. Cell disruption was achieved by three cycles of freezing the cell suspension in liquid nitrogen followed by thawing at 30°C. After sonication of the suspension (3 x 10 sec), the insoluble material was removed by centrifugation at 20,000 g for 30 min at 4°C. The cleared supernatant was supplemented with 20 µg/ml DNaseI and incubated 45 min on ice to digest the genomic DNA. The solution was cleared through a 0.45 µm low-protein binding Millex-HV filter (Millipore, Bedford, USA) and degassed. 5 mM DTT were added to the soluble extract and proteins were directly applied at 1 ml/min flow rate to a 5 ml HiTrap Chelating HP column charged with Ni2+. Unspecific bound protein was washed with 50 mM sodium phosphate pH 7.4, 500 mM NaCl, 10 mM imidazole, 5 mM DTT until no further protein eluted. Bound protein was eluted with a 100 ml linear gradient to 50 mM sodium phosphate 7.4, 500 mM NaCl, 500 mM imidazole, 5 mM DTT and fractions containing rArt v 1 were pooled.

### Proteolytic cleavage of histidine-tagged fusion proteins

Purified 6x-histidine tagged Art v 1 proteins were digested with recombinant protease of tobacco etch virus (rTEV) to remove the histidine tag from the protein. Recombinant TEV, which was produced in the laboratory, displays a poly histidine tag, which allows one-step purification subsequently to the proteolytic digest. The fusion protein was directly digested in the buffer obtained during protein elution in the IMAC purification (50 mM sodium phosphate pH 7.4, 500 mM NaCl, 120-160 mM imidazole). Recombinant TEV was added in a weight ration of 1:2 to 1:4 (rTEV/histidine tagged protein) and incubated for 24 hours at 29°C. The proteins were briefly dialyzed against dH20 and supplemented with 10x buffer to achieve 50 mM sodium phosphate pH 8.0, 500 mM NaCl, 10 mM imidazole, and 5% (v/v) glycerol. To remove cleaved histidine tag, uncleaved protein and rTEV the sample was loaded on a 5 ml HiTrap Chelating HP column using the ÄKTAprime system. Fractions containing protein with apparent purity >95% were pooled, dialyzed against dH20 and lyophilized in aliquots.

### Immunoblots

Sera or plasma from atopic and non-atopic individuals were diluted 1:10 in 50 mM sodium phosphate buffer pH 7.4, 0.5 % (v/v) Tween-20, 5 % (w/v) bovine serum albumin, 0.05 % (w/v) sodium azide. Immunoblots were incubated with diluted patient's sera over night at 4°C. Bound IgE antibodies were detected with 125I-rabbit anti-human IgE on a phosphorimager screen using a Fujifilm BAS-1800 II reader (Fuji, Japan). Alternatively, autoradiography was performed at -70°C with intensifying screens (KODAK, Rochester, NY, USA).

### Amino acid analysis

Amino acid composition of purified recombinant proteins was determined using the Pico-Tag method (Waters, Milford, MA, USA). 10 - 15 µg lyophilized protein was reconstituted in ddH20 and dried under vacuum in 6 x 50 mm glass tubes using a Pico-Tag workstation. Protein samples were hydrolyzed with constant boiling 6 M HCl for 16 - 18 hours at 121°C under vacuum. After neutralization with aqueous 20% (v/v) triethylamine, 40% (v/v) ethanol, protein samples and amino acid standards (Pierce, Rock-ford, IL, USA) were re-dried. Derivatization of the amino acids was performed for 10 min at room temperature using 10% phenylisothiocyanate (v/v) as derivatizing agent in solutions of 70% (v/v) ethanol and 10% (v/v) triethylamine. Dried samples were resuspended in 200 µl Pico-Tag Eluent A, prepared according to the recipe provided by the Pico-tag manual. Phenylthiocarbamyl amino acid derivatives were analyzed by reversed phase-HPLC (HP 1100, Agilent, Technologies, Palo Alto, CA, USA). Chromatography was performed at 38°C using a 3.9 x 150 mm, 4 µm Nova-Pak C18 column (Waters, USA) with an acetonitrile gradient according to the protocol. UV absorbance was monitored at 254 nm and amino acid composition was calculated based on the specified amount in the amino acid standard. Cysteine and tryptophan were not analyzed using this standard protocol for amino acid analysis.

### Reduction and alkylation

70 µg of purified rArt v 1 and three rArt v 1-cysteine variants were dissolved in 0.3 M Tris-HCl pH 8.0, 8 M guanidinium-HCl, 1.3 mM EDTA to a final concentration of 1 µg/µl. For reduction of the disulfide bonds, DTT was added to the protein in a 50-fold molar excess (2 µmol) and incubated 4 hours at 37°C. Alkylation was performed by the addition of 4 µmol freshly dissolved iodoacetamide and incubation for 1 hour at 37°C. After stopping the reacting with 2 µmol DTT the solution was extensively dialyzed against dH2O.

### Proteolytic digests

Reduced and alkylated proteins were dissolved to 1 µg/µl in ddH20. After denaturation at 95°C for 20 min samples were immediately cooled on ice and supplemented with ammonium bicarbonate buffer pH 7.8 to a final concentration of 100 mM. Proteins were digested with trypsin and Glu-C sequencing grade (Boehringer Ingelheim, Ingelheim, Germany). Endoproteinases were added to the proteins in a ration of 1:30 by weight. Proteolytic digests were incubated at 37°C overnight and the reactions were stopped by the addition of 1/10 volume trifluoroacetic acid and lyophilized.

### Mass analysis

Data were acquired using electrospray ionization coupled to a Quadrupole Time of Flight Mass Spetrometry (ESI-QTOF MS) (Micromass QTof Global Ultima mass spectrometer, Waters, Milford, MA, USA). Dried protein samples were dissolved in aqueous 50% (v/v) HPLC-grade acetonitrile in 0.1% (v/v) formic acid at a concentration of approximately 100 fmol/µl and directly infused at a rate of 5 µl/min. Typically, spectra of positively charged ions were recorded for 3 to 5 minutes and combined. The multiply charged spectra for obtaining the molecular mass of intact proteins were deconvoluted with the Micromass MaxEnt1 software. Proteolytic fragments were analyzed using the MaxEnt3 software (Waters, Milford, MA, USA).

### Circular dichroism

Circular dichroism (CD) spectra were recorded in aqueous solutions with a JASCO J-810 spectropolarimeter (Jasco, Essex, UK) fitted with a Neslab RTE-111M temperature control system (Thermo Neslab Inc., Newington, NH, USA). Measurements were performed with a quartz cuvette of 0.1 cm pathlength. Far UV CD spectra were recorded at 20°C, with a bandwidth of 1 nm, a response time of 1 sec and a data pitch of 1 nm. Measurement range was between 190 - 260 nm and obtained spectra represent an average of 5 consecutive scans. All measurements were performed using 10 mM sodium phosphate pH 7.3 as buffer system. The working protein concentration was 0.1 µg/µl, and was determined for each protein by amino acid analysis. All data are presented as mean residue molar ellipticity [Θ]MRW at a given wavelength taking protein concentration, molecular weight and the number of residues into account. Thermal denaturation of each protein was monitored in the temperature range of 25 to 95°C at a fixed wavelength of 203 nm. The temperature slope was set at 1°C/min and full CD spectra were recorded from 25°C to 95°C every 10 degrees using the parameters listed above. The reversibility of the unfolding process was determined by monitoring a full CD spectrum after cooling the denatured protein at 1°C/min to 20°C.

### ELISA and cross-inhibition experiments

Mugwort pollen extract, purified nArt v 1, rArt v 1, and three variants were diluted to 5 µg/ml in PBS pH 7.4 and 50 µl per well were coated to Maxisorp ELISA plates (NUNC, Roskilde, Denmark) overnight at 4°C. Plates were washed twice with TBS pH 7.4, 0.05% Tween-20, and blocked with TBS pH 7.4, 1% BSA, 0.05% Tween-20 for 4 hours at room temperature. Patients' sera were diluted 1:4 in TBS pH 7.4, 0.5% BSA, 0.05% Tween-20 and incubated overnight at 4°C. After 5 times washing with TBS pH 7.4, 0.05% Tween-20, plates were incubated with alkaline phosphatase conjugated monoclonal anti human IgE antibodies (BD Biosciences PharMingen, San Diego, CA, USA), 1:2000 diluted in TBS pH 7.4, 0.5% BSA, 0.05% Tween-20 and incubated 1 hour at 37°C followed by 1 hour at 4°C. After 5 times washing as described above 100 µl 10 mM 4-Nitrophenylphosphate dissolved in 0.5 M diethanolam-in-HCl pH 9.8, were added and incubated for 20 to 60 min at room temperature. Optical density of the color reaction was read at 405 nm wavelength (reference 492 nm) in a Dynatech MR 600 ELISA reader (Dynex Technologies, Chantilly, VA). All measurements were performed as duplicates, background values were subtracted and results are presented as mean values thereof.

For cross-inhibition experiments purified natural or recombinant Art v 1 was coated to Maxisorp ELISA plates and washed as described above. Five atopic patients' sera previously shown to react with nArt v 1 and rArt v 1 were used for this study. Sera were diluted 1:3 in TBS pH 7.4, 0.5% BSA, 0.05% Tween-20 and pre-incubated with increasing amounts of inhibitor proteins. All purified Art v 1 proteins and variants were used at concentrations of 0, 0.002, 0.02, 0.2, 2, 20 and 200 µg/ml to bind patients' IgE overnight at 4°C. Incubation, washing and detection of bound IgE were completed as described above. The percentage of IgE inhibition after antigen pre-incubation was calculated as: 100-(OD with inhibitor x 100/OD without inhibitor).

### Rat basophil leukemia cell mediator-release assay

Rat basophil leukemia (RBL) cells, subline RBL-2H3 were transfected with human FcεRI receptor to bind human IgE. Upon antibody binding degranulation of the RBL, cells can be provoked by IgE crosslinking leading to β-hexosaminidase release. The relative enzymatic acitivity of β-hexosaminidase in the culture supernatant can be quantified by colorimetric reaction. For the experiments transfected RBL cells were coated with serum IgE from 6 mugwort pollen-allergic patients, stimulated with allergen and degranulation was monitored. To ensure reproducibility of the system and quality of the cells, transfected RBL cells were sensitized with polyclonal human IgE (Biogenesis, Poole, England) and with mouse IgE (culture supernatant of the hybridoma cell line IGEL.b4). Cells were degranulated using specific anti IgE antibodies, goat anti human IgE GaHIgE/Fc/7S (Nordic Immunologic Laboratories, Tilburg, The Netherlands) for the human system and alternatively goat anti mouse IgE GAMIgE/Fc/7S (Nordic Immunologic Laboratories, Tilburg, The Netherlands) for mouse IgE crosslinking. RBL cells for applications with human IgE were cultured in Eagle's MEM / RPMI (80:20) supplemented with 5% inactivated fetal calf serum, 1 g/l geneticin sulphate (G-418). Eagle's MEM / RPMI was not supplemented with geneticin sulphate when used with mouse IgE specific cell lines.

For sensitization with human antibodies, cells were harvested in the stationary phase and 50 µl aliquots (2 x 10⁶ cells/ml) were plated in 96-well tissue culture plates (Nunc, Roskilde, Denmark). Human IgE or sera were diluted 1:20 and 50 µl aliquots were administered to the cells and incubated overnight at 37°C, 5% CO₂. Alternatively, 100 µl aliquots of RBL cells (2 x 10⁶ cells/ml) were attached to 96-well tissue culture plates overnight at 37°C, 5% CO₂. The medium was replaced by 100 µl fresh Eagle's MEM /RPMI supplemented, mouse IgE was added followed by an incubation step for at least 1 h at 37°C, 5% CO₂. After passive sensitization, culture media containing IgE was removed and the cell layer washed 3 times with Tyrode's buffer (130 mM NaCl, 5 mM KCl, 1.4 mM CaCl₂, 1 mM MgCl₂, 5.6 mM glucose, 10 mM HEPES, 0.1% BSA, pH 7.4). For assays with human IgE Tyrode buffer was composed with 50% D₂O (Sigma-Aldrich, Vienna, Austria). Spontaneous release (= 0%) was measured with 100 µl Tyrode buffer without allergen or anti IgE antibodies, total release (= 100%) was obtained by addition of 100 µl 1% Triton X-100 (Sigma-Aldrich, USA). Triggering of RBL cells was induced by addition of 100 µl Tyrode buffer per well plus allergens added in serial dilutions. Cells were incubated for 1 hour at 37°C, 5% CO₂. 30 µl supernatant were transferred to a fresh 96-well plate and mixed with 50 µl p-nitrophenyl-N-acetyl-β-D-gluc-osaminide (1.3 mg/ml in 0.1 M Na₂HPO₄ buffer, pH 4.5 adjusted with citric acid). After incubation for 1 hour at 37°C the reaction was terminated by adding 100 µl 2 M glycine pH 10.7 and activity of released β-hexosaminidase was measured at 405 nm (reference 620 nm). The percentage of mediator release was calculated as proportion of total release after subtracting the value for spontaneous release.

### RESULTS

### Expression and purification of rArt v 1 and three variants

Recombinant Art v 1 and cysteine variants C22S, C47S, and C49S were expressed in E. coli strain BL21-CodonPlus(DE3)-RIL as 6x-histidine tagged fusion proteins. This E. coli strain encodes additional tRNA's and was used to improve the expression of soluble Art v 1 proteins and variants thereof. Several conditions for the production were tested to optimize the expression of the recombinant Art v 1 proteins. Growth of bacteria at temperatures higher than 26°C resulted in large amounts of C-terminally degraded protein. Therefore, protein expression was generally performed at 16°C overnight to minimize degradation of Art v 1 proteins, which was shown to arise during protein translation. To overcome the growth problems of the expression culture, induction with IPTG was performed when the bacterial culture had already reached the stationary phase (OD₆₀₀ 1.6-1.8).

Another expression strategy was used to enhance the yield of recombinant Art v 1 under bacterial stress conditions. Prior to protein expression, the bacterial culture was supplemented with 4% NaCl, 0.5 M sorbitol and grown under osmotic stress in the presence of compatible solutes (glycine betaine). This low-molecular weight osmolyte naturally occur in halophilic bacteria and are known to protect proteins at high salt concentrations. Expression of recombinant Art v 1 in this high-salt environment resulted in the accumulation of 2-3 times more protein than obtained under normal expression conditions.

Using pHIS-parallel2 expression vector, three residual amino acids (G-A-M) remained fused to the N-terminus of recombinant Art v 1 and the three variants. The wild type Art v 1 protein preparation containing 3 extra amino acids was compared with a different batch of recombinant Art v 1 produced as non-fusion protein in E. coli. No differences were observed in the reactivity with rabbit antibodies raised against Art v 1 or patient's IgE. Therefore the influence of these three extra residues on the allergenic or antigenic behavior of the proteins was considered non-relevant.

10-20 mg recombinant Art v 1, variant C22S, C47S and C49S were obtained from bacterial expression in E. coli. Purity of all recombinant allergens was demonstrated to be at least 95%. All preparations of recombinant protein showed a single prominent band at an apparent molecular weight of 19 kDa (theoretical 11 kDa). The unusual migration pattern of Art v 1 proteins in SDS-PAGE was described earlier for recombinant as well as natural Art v 1. A faint additional band migrating at approximately 35 kDa in the preparation of C22S, C47S and C49S presumably represents a dimer of the recombinant proteins (Fig. 5).

### Amino acid composition

Amino acid analysis was performed for recombinant Art v 1 and cysteine variants C22S, C47S and C49S using the Pico-tag method and the observed amino acid contents of all analyzed samples were in excellent correlation with the theoretical composition. The exact concentration of all protein preparations was determined using the amino acid standard with known concentration of all amino acids as calculation base.

### ESI-QTOF mass spectrometry

Purified recombinant Art v 1, variants C22S, C47S, and C49S were reduced and alkylated prior to mass determination. All masses obtained were in good correlation with the calculated values. Successful in vitro cleavage of all recombinant proteins produced as histidine tagged fusion-proteins was confirmed with no observable post-translational modifications or amino acid alterations.

Fragments obtain from proteolytic digests with trypsin and Glu-C were used to confirm the primary structure of recombinant Art v 1 and mutant proteins. Complete sequence identification was achieved for recombinant Art v 1, C22S and C47S with fragments obtained from both proteolytic digests. The sequence of the cysteine variant C49S was covered to 95% as one fragment of 5 amino acids was not identified in mass analysis.

### Circular dichroism (CD) spectroscopy

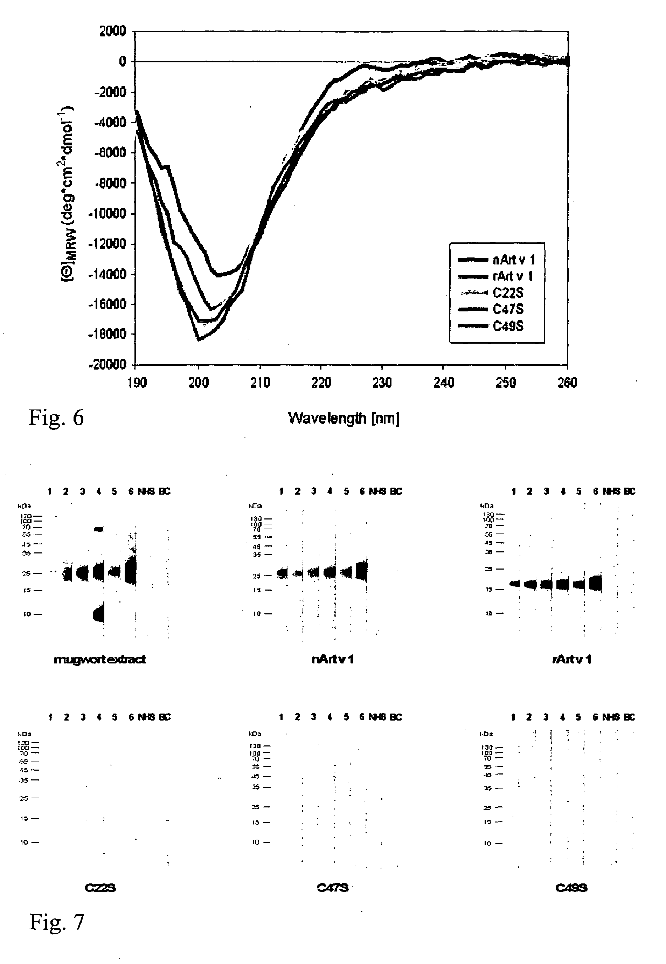

As previously determined by computer modeling of Art v 1, the C-terminal domain of the proteins was assumed to show a polyproline left-handed helical structure. These polyprolines show no alpha-helical, beta-turn or beta-sheet structure and were in the past often confused with unordered or random coil. Circular dichroism spectra obtained for all Art v 1 proteins did resemble a typical polyproline curve. Most likely the signals of the 21 prolines or hydroxyprolines (in case of the natural Art v 1) in the C-terminal domain of Art v 1 seem to overlap the secondary structures presumably present in the N-terminal defensin domain. Natural Art v 1, which was purified under mild conditions from mugwort pollen extract, also shows this typical spectrum as was demonstrated before. The CD curve of nArt v 1 showed a reduced peak amplitude at 203 nm compared to all recombinant proteins and slightly different values were obtained in the range of 220 - 230 nm. Similar spectra were derived for the Art v 1-cysteine mutants; the spectrum of rArt v 1 was located rather between the curve of nArt v 1 and the rArt v 1 variants. Although slight differences in the CD spectra of natural, recombinant Art v 1 and the three cysteine variants were observed, the overall secondary structure seems to be rather similar. Thus, the folding of Art v 1-cysteine mutants resembles those of the natural glycosylated and recombinant non-glycosylated Art v 1 proteins (Fig. 6).

Thermal denaturation of all proteins was performed using a temperature gradient from 25°C to 95°C and changes in CD signals were recorded at 203 nm (representing the negative amplitude). Determination of the melting temperature was not possible, but denaturation clearly demonstrated a change in the shape of the curves. The ability to refold upon heating the proteins to 95°C was shown for nArt v 1, as the curve was completely identical with the spectrum obtained prior to denaturation. All recombinant Art v 1 proteins were able to partially refold after thermal denaturation.

### IgE reactivity

The capacity of mugwort pollen extract, nArt v 1, rArt v 1 and three mutants to bind IgE from Art v 1-allergic patients was tested in immunoblot experiments. Purified proteins and mugwort pollen extracts containing equivalent amounts of nArt v 1 were separated by SDS-PAGE under reducing conditions and blotted onto nitrocellulose. Sera from mugwort pollen-allergic patients (n=6), previously shown to react with natural and recombinant Art v 1 were used to demonstrate IgE reactivity with the Art v 1 proteins. All sera tested showed strong reactivity with natural Art v 1 (in the mugwort pollen extract or purified protein) and recombinant Art v 1 in the immunoblot experiment. No signal was obtained for variant C22S, C47S, and C49S of Art v 1 with any of the sera tested. These results demonstrate the low IgE-binding activity of the three mutants C22S, C47S, and C49S (Fig. 7).

### ELISA

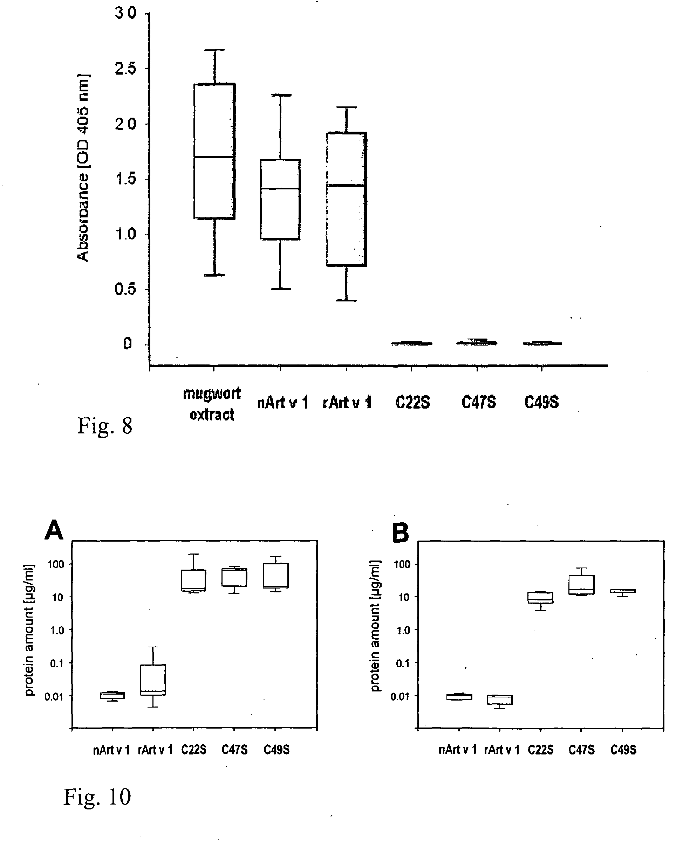

The IgE binding capacity of recombinant Art v 1, variants C22S, C47S and C49S was determined by ELISA using patients' sera derived from 16 mugwort pollen-allergic patients. Average IgE reactivity of natural and recombinant Art v 1 was observed to be the same for these experiments, as patient's with similar IgE reactivity to both proteins were pre-selected. 3 of the 16 patients' sera showed higher reactivity to recombinant Art v 1 and other 3 showed increased reactivity to natural Art v 1. Slightly higher IgE reactivity was obtained for the mugwort pollen extract as some sera were also reacting with other minor mugwort pollen allergens. The three hypoallergenic variants of Art v 1 showed a dramatically reduced IgE reactivity compared to natural Art v 1 (P < 0.001) as well as recombinant Art v 1 (P < 0.001). Average relative IgE reactivity to the cysteine variants was 100-200 times reduced in ELISA experiments compared to natural and recombinant Art v 1. Incubation with non-atopic serum showed no reactivity with any of the proteins tested in the ELISA assay and reactivity of allergen derivatives with antibodies used for detection of bound serum IgE was excluded experimentally (Fig. 8).

### Cross-inhibition ELISA

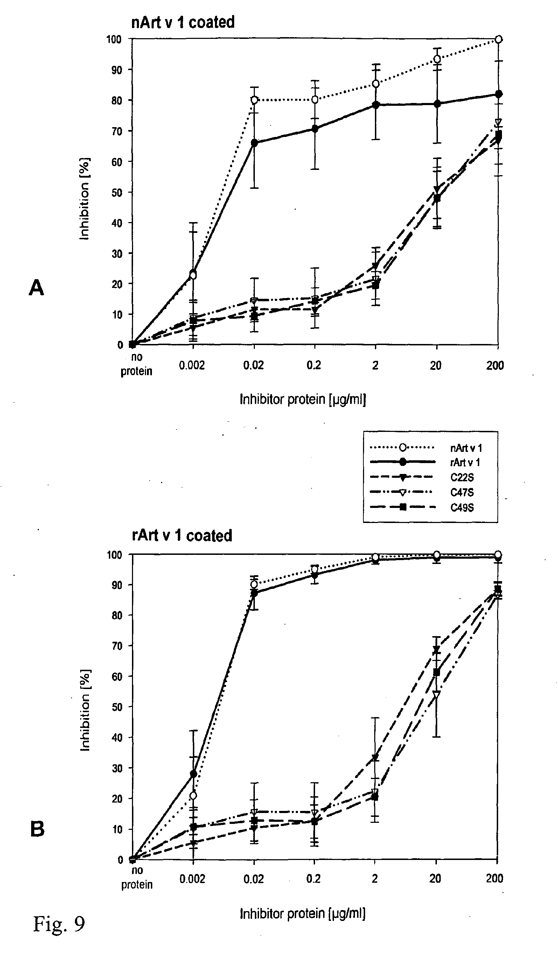

Quantitative IgE inhibition experiments were performed with plasma derived from 5 patients, previously shown to react with natural and recombinant Art v 1 in ELISA studies. Complete inhibition was observed using natural Art v 1, which was able to inhibit immobilized nArt v 1 as well as rArt v 1 at very low concentrations. Recombinant Art v 1 completely inhibited IgE binding to immobilized rArt v 1, but showed only 65% to 90% inhibition of binding to nArt v 1 at the highest inhibitor concentration used (200 µg/ml). These values varied considerably among the 5 different patients' sera. Especially one serum reacted significantly weaker with rArt v 1 compared to nArt v 1 in previous ELISA studies and was not able to be efficiently blocked by recombinant Art v 1. Extremely reduced potency to inhibit natural as well as recombinant Art v 1 was found for all cysteine variants. At low inhibitor concentrations ranging from 0.002 to 0.2 µg/ml only minor inhibition (up to 20%) was observed for C22S, C47S and C49S in contrast to the very potent inhibitors natural and recombinant Art v 1 at these concentrations (70-90%). At the highest concentration used (200 µg/ml), the cysteine variants of Art v 1 showed an inhibitory capacity up to 70% for coated nArt v 1 and up to 90% for coated rArt v 1 (Fig. 9).

Protein amounts that reached 50% inhibition of either coated natural or recombinant Art v 1 were calculated for each individual inhibitor protein and patient. Similar mean protein amounts of nArt v 1 and rArt v 1 were necessary to reach the 50% inhibition value (0.009-0.01 µg/ml and 0.008-0.067 µg/ml, respectively). Ten-fold higher levels of rArt v 1 protein were needed to block patient's IgE binding to coated natural Art v 1. Absolute protein amount for 50% inhibition was 9.52 and 52.45 µg/ml for C22S, 30.54 and 50.29 µg/ml for C47S, 15.06 and 60.47 µg/ml for C49S; first value indicates inhibition of coated nArt v 1, and the second value corresponds to coated rArt v 1. All three low IgE-binding variants of recombinant Art v 1 revealed statistically different values compared to natural (P = 0.008) and recombinant Art v 1 (P = 0.008). An increase of 1,000 to 7,000 fold in protein concentration necessary to achieve 50% inhibition was observed for mutant C22S, C47S, and C49S compared to nArt v 1 and rArt v 1 (Fig. 10).

### Degranulation assay of rat basophil leukemia cells

The allergenic potential of wild-type Art v 1 and mutants was determined by degranulation of rat basophil leukemia (RBL) cells using sera of 6 individual mugwort pollen-allergic patients. To assess dose dependency of basophil degranulation upon stimulation with Art v 1 allergens, transfected RBL cells were challenged with serial dilutions of purified nArt v 1, rArt v 1 and three cysteine variants. Allergen-dependent maximal mediator release was observed with natural Art v 1, but release values did substantially vary among the individual patients. Results from the first RBL release experiments showed that the highest allergen dilution of nArt v 1 and rArt v 1 did already result in substantial release of mediator when compared to the C22S, C47S, and C49S cysteine variants, which could not trigger a mediator release at the lower concentrations. To corroborate these data, the experiment was repeated with minor modifications; the highly reactive allergens (i.e. nArt v 1 and rArt v 1) were further diluted stepwise to 10-9 µg/ml. Natural and recombinant Art v 1 were not tested in the higher concentration range, as was already demonstrated in the last experiment that saturation of the curve is already achieved at a concentration of 1 ng/ml. Natural Art v 1 induced the highest release values; whereas recombinant Art v 1 was only able to trigger similar response to nArt v 1 in two patients. In general release curves for nArt v 1 and rArt v 1 were quite similar with regard to the onset of mediator release, but maximal values differed as recombinant Art v 1 could only elicit 50-70% of the maximum release achieved for nArt v 1 in four of the patients' sera tested. Variants C22S, C47S, and C49S showed completely different response curves, especially regarding the onset of mediator release. These three hypoallergenic mutants were able to trigger a considerable β-hexosaminidase release only at the highest protein concentrations used (0.1-10 µg/ml). In contrast to natural and recombinant Art v 1, no mediator release activity could be observed at low concentration of the variants (Fig. 11).

Half-maximal amount of the highest release value obtained from IgE cross-linking with nArt v 1 was calculated for every individual patient's serum. Corresponding concentrations were calculated for all allergens to provoke half-maximal basophil degranulation and mean amounts thereof were determined. Lowest amounts of protein were necessary in case of nArt v 1 (0.00007 µg/ml) to trigger the release, followed by recombinant wild type Art v 1 (0.0004 µg/ml) which was about 5 times less potent (P = 0.026) than natural Art v 1. Mean values of the hypoallergenic variants were determined to be 6.5 µg/ml for C22S, 7.7 µg/ml for C47S, and 6.1 µg/ml for C49S in triggering half maximal releases. Statistical significant differences to nArt v 1 (P = 0.002) and rArt v 1 (P = 0.002) were observed for all three mutants. Differences in relative protein amount for the cysteine variants compared to nArt v 1 were determined to be about 100,000 fold higher to provoke a similar release. Comparison of the mutants with rArt v 1 resulted in protein amounts about 50,000 fold higher than observed for the recombinant protein. It was shown again that C22S, C47S and C49S possess low IgE binding activity as was demonstrated before in the ELISA experiments. These results highlight the hypoallergenic character of the C22S, C47S and C49S cysteine variants of Art v 1, as these in vitro experiments resemble the in vivo biological activity of the allergens in an allergic reaction.

### 1.5. Cloning of Art v 1-defensin and -proline domains

In the experiments under item 1.5. the role of the defensin and proline-rich domains of Art v 1 in antibody recognition were investigated.

### Methods

The wild-type sequence corresponding to Art v 1-defensin and the proline domains were cloned into the pHIS-parallel2 expression vector using a 5'NcoI and 3'*XhoI* restriction site. The following primers were used: DEF fwd *Nco*I: 5'-GAGAGACCATGGCTGGTTCAAAGTTG-3' (SEQ ID No. 44); DEF rev *Xho*I: 5'-GAGAGACTCGAGT-TACGATTTGGAACAGTCAAAGTA- 3' (SEQ ID No. 45); PRO fwd *Nco*I: 5'-GAGAGACCATGGCCTCCTGGAGCAACACCAGCG-3' (SEQ ID No. 46) and PRO rev *EcoR*I: 5'-GAGAGACTCGAGTTAGTGAGTGGACGGAGGAGG-3' (SEQ ID No. 47). Obtained DNA fragments were ligated into the respective sites of pHIS-parallel2 vector using standard molecular biology techniques. All constructs were sequenced according to the Dye Terminator Cycle Sequencing protocol.

### Results

Two expression constructs, i.e. Art v 1-defensin domain (amino acid 1-56) and Art v 1-proline domain (amino acid 57-108) in pHIS-parallel2 for expression of histidine tagged proteins were generated.
Amino acid sequence of Art v 1-defensin domain:
AGSKLCEKTSKTYSGKCDNKKCDKKCIEWEKAQHGACHKREAGKESCFCYFDCSKS (SEQ ID No. 48)
Amino acid sequence of Art v 1-proline domain
PPGATPAPPGAAPPPAAGGSPSPPADGGSPPPPADGGSPPVDGGSPPPPSTHG (SEQ ID No. 49)

### Expression, purification and characterization of Art v 1-defensin and proline domains

### Methods

The constructs were transformed into *E. coli* BL21-CodonPlus(DE3)-RIL cells for protein expression. Several clones of the transformation plate were used to inoculate liquid SB medium with 100 mg/l ampicillin and grown at 37°C overnight. The pre-culture was diluted 1:30 in fresh, pre-warmed media and cells were grown at 37°C to an OD₆₀₀ of 0.8. IPTG was added to a final concentration of 1 mM, and incubation continued for 4 hous at 37°C. Bacterial cells were harvested by centrifugation at 4,500 g for 15 min at 4°C and stored at -20°C.

Protein purification of the defensin domain was performed as was described above in detail for the recombinant Art v 1 and three selected variants, followed by cleavage of the 6xhistidine tag using rTEV protease. Finally the protein was dialyzed against water, lyophilized and stored at -20°C.

The Art v 1-proline domain was purified under denaturing conditions including 6 M urea in all buffers for the immobilized metal affinity chromatography. Purified protein was dialyzed against phosphate buffer including 4 M urea and stored at 4°C. The protein preparations were characterized regarding physicochemical and immunological properties (as described above for Art v 1, C22S, C47S, and C49S).

### Results

Expression of recombinant Art v 1-defensin domain resulted in approximately 10 mg/liter of protein purified from the soluble fraction. Production of the defensin domain alone compared to the full-length protein was found to be superior, as the yield of recombinant protein was 2-3 folds higher and degradation products were not abundant in this preparation. Thus, from the production point of view, the defensin domain is definitely better than Art v 1 full-length protein and mutants. As the immunological properties of the proline domain produced in bacteria seems to be irrelevant, and the defensin domain is comparable to the full-length protein with respect to all tests performed, the production of this domain or mutants thereof would be preferred.

The purified protein was extensively characterized and the primary structure was confirmed using amino acid and mass analysis. The Art v 1-proline domain was extracted from inclusion bodies and was purified under denaturing conditions. Refolding of the protein was not successful, and therefore only limited investigations could be performed with the proline domain, as it had to be stored in 4 M urea.

Immunological characterization using immunoblot experiments, ELISA, cross-inhibition ELISA, and rat basophil histamine release experiments revealed a striking difference between the two domains of Art v 1. The Art v 1-defensin domain demonstrated the same immunological properties as full-length recombinant Art v 1 in all experiments, suggesting that all relevant IgE binding epitopes of the recombinant protein are located in the N-terminus of the allergen (Figs. 12 and 13). In contrast, the Art v 1-proline domain showed no immunological reactivity, which could be attributed to lack of proper folding. Production of the proline domain in bacteria might not be appropriate to obtain a correctly folded protein, and plant expression systems should be exploited. Additionally, natural Art v 1 was shown to contain all O-glycans at the C-terminal proline domain, which could also be a prerequisite for the stability of this extended domain.

### 1.6. Cloning of single and double point mutants of Art v 1-defensin domain

### Methods

Four single point defensin mutants were generated using the Art v 1 C22S, C26S, C47S, and C49S as template for amplification of the N-terminus of the protein. The following primers were used for cloning into the pHIS-parallel2 expression vector: DEF fwd *Nco*I: 5'-GAGAGACCATGGCTGGTTCAAAGTTG-3' (SEQ ID No. 44) and DEF rev *Xho*I: 5'-GAGAGACTCGAGTTACGATTTGGAACAGTCAAAGTA-3' (SEQ ID No. 45).

Two double mutants (C22S-C47S and C26S-C49S) were obtained using DEF fwd *Nco*I (listed above) and DEF C47S rev *Xho*I: 5'-GAGACTCGAGTTACGATTTGGAACAGTCAAAGTAGCAAAGGAACTT-3' (SEQ ID No. 50) or DEF C49S rev *Xho*I: 5'-GAGACTCGAGTTACGATTTGGAACAGT-CAAAGTAGGAAAAG-3' (SEQ ID No. 51) to introduce an additional point mutation. Obtained DNA fragments were ligated into the respective sites of pHIS-parallel2 vector and constructs were sequenced according to the Dye Terminator Cycle Sequencing protocol.

### Results

In total six different cysteine variants of the Art v 1-defensin domain were constructed. All positions that were experimentally shown to be important for serum IgE reactivity of Art v 1 were included in this approach. Thus, single point mutants C22S, C26S, C47S, and C49S and double mutants C22S-C47S and C26S-C49S of the Art v 1-defensin domain were generated.

### Example 2: Allergen-specific T cell responses to Art v 1, defensin-domain of Art v 1 and their variants

At present, the only causative therapy of type I allergy is specific immunotherapy (SIT). Conventional SIT consists of repeated subcutaneous injections of increasing amounts of allergen extracts over a long time period. SIT is effective in selected patients and provides a long-term tolerance to the allergen based on alterations in the antigen-specific immune response. However, besides the difficulty of standardizing complex allergenic mixtures, the administration of allergen extracts bears the risk of inducing IgE-mediated anaphylactic side effects and potential sensitization to additional allergens contained in the vaccine. A strategy to increase the safety and efficacy of SIT is the development of modified allergens with decreased ability to bind allergen-specific IgE (hypoallergens). Since T cells play a major role in the desensitization process, it is necessary to retain the relevant T cell epitopes in these hypoallergenic variants.

### 2.1. Characterization of the T cell responses to Art v 1

The T cell response to Art v 1 was characerized in detail using an *in vitro* culture system, which makes use of allergen-specific T cells in peripheral blood mononuclear cells (PBMC), oligoclonal T cell lines (TCL) and monoclonal T cell clones (TCC). These cultures can be phenotypically and functionally characterized for surface marker expression, proliferative responses, cytokine production, T cell receptor (TCR) usage, HLA-restriction and T cell epitope recognition.
Comparing the proliferative responses induced by natural (n)Art v 1 and recombinant (r)Art v 1 in PBMC, antigen-specific TCL and TCC, a similar potency was observed indicating that the carbohydrate moiety of Art v 1 plays no obvious role in T-cell activation. Mugwort allergic patients showed a typical Th2-dominanted T-cell response to Art v 1 (Himly et al., FASEB J. 17, 106-108,2002; Jahn-Schmid et al., J Immunol.169:6005-11, 2002). However, in striking contrast to other allergens which typically contain multiple T-cell epitopes, Art v 1 harbours only one single immunodominant epitope (Art v 1₂₅₋₃₆) which is located in the defensin domain (Fig. 14) (Jahn-Schmid et al., J Immunol. 169:6005-11, 2002). In addition, the core of T cell activating region within Art v 1₂₅₋₃₆ was characterized and amino acid residues essential for T cell stimulation were determined (Jahn-Schmid et al., J Allergy Clin Immunol 115:399-404, 2005).

Another very unusual feature of mugwort pollen allergy is the fact that mugwort pollen allergy is highly associated with the HLA-DRB1*01 phenotype (p<10⁻⁶) (Jahn-Schmid et al., J Allergy Clin Immunol 115:399-404, 2005). CD4+ T cells recognize linear peptides that arise during antigen processing in the context of HLA class II molecules on antigen-presenting cells. These molecules are extremely polymorphic, i.e. they can be encoded by a multitude of allelic genes. It was demonstrated that HLA-DRB1*01 is especially effective in antigen-presentation of the immunodominant peptide of Art v 1. Only a few and by far less strong associations of HLA class II antigens with responses to major allergens have been reported so far.

Art v 1 and the following hypoallergenic full-length Art v 1-mutants, the defensin-domain or mutants thereof were tested *in vitro* for their capacity to activate Art v 1-specific T lymphocytes to demonstrate that the relevant T cell epitopes were retained:
**Art v 1 C22S (C22S)**
**Art v 1 C47S (C47S)**
**Art v 1 C49S (C49S)**
**Defensin-domain wild type (DefWT)**
**Defensin-domain C22S (DefC22S)**
**Defensin-domain C26S (DefC26S)**
**Defensin-domain C47S (DefC47S)**
**Defensin-domain C49S (DefC49S)**

### Methods:

### Antigen-reactive T lymphocytes

PBMC were isolated from heparinized blood by Ficoll-density gradient centrifugation. For primary *in vitro* responses 2x10⁵ PB-MC/well were stimulated with allergens at different concentrations (geometric dilution from 20µg/ml) for 7 days in serum-free medium at 37°C and 5% CO₂. Proliferation was assessed by measuring uptake of ³H-thymidine (0.5 µCi/well) during the last 16 hours, harvesting the cells onto glass fiber mats, followed by liquid scintillation counting. TCL and TCC were obtained from PBMC as follows: 1.5 x 10⁶ PBMC were stimulated with 4 µg/ml of mugwort extract (TCL MW) or 10 µg/ml purified natural or recombinant Art v 1 (TCL nat/rec) in 24-well flat-bottomed culture plates. After 5 days suboptimal doses of human rIL-2 were added and cultures were continued for additional 7 days. Then, T cell blasts were enriched by density centrifugation with Ficoll. The majority of T cell blasts was expanded with IL-2 and irradiated allogeneic feeder (PBMC from a different donor individual) for T cell stimulation experiments. Some of the blasts were used to establish monoclonal T cell cultures by limiting dilution: 0.3 cells/well from Art v 1-specific TCL were seeded into 96 well round bottom plates in the presence of 2 x 10⁵ irradiated allogeneic PBMC as feeder cells, 0.25 % v/v PHA and rIL-2 (4 U/well) in the medium mentioned above. After 14-21 days, growing microcultures were expanded at weekly intervals with fresh irradiated allogeneic PBMC and rIL-2. The specifity of TCL/TCC was assessed in proliferation assays as soon as the cell number reached 2x10⁵. Proliferation assays were performed using 2x10⁴ T cells and 1x10⁵ autologous or HLA-matched, irradiated (60Gy) PBMC or 4x10⁴ EBV-transformed B-cell lines as antigen-presenting cells. After 48 hours thymidine uptake was measured. When the stimulation index (SI; ratio between proliferation (measured in counts/min (cpm) obtained in cultures containing TCC plus autologous antigen-presenting cells (APC) plus antigen, and cpm obtained in cultures containing TCC and APC alone) was >10, responses were considered as positive. For PBMC and TCL, which showed different degrees of background proliferation due to autoreactivity, a SI >3 or respectively 10.000 dpm (delta cpm = proliferation with allergen in cpm - background proliferation with medium alone in cpm) were considered as cut-off for antigen-specificity. Art v 1-specific TCL/TCC were expanded by alternating turns of stimulation with autologous irradiated APC and Art v 1 or with allogeneic feeder cells and rIL-2.

### Measurement of cytokines

TCL/TCC were washed and incubated with irradiated autologous APC in the presence of Art v 1 (5 µg/ml) for 24 hours. Cytokine levels in the resulting supernatants were measured in ELISA using matched Ab pairs (Endogen, Woburn, MA) according to instructions by the manufacturer (sensitivity limits: IL-4: 9 pg/ml, IFN-γ: 9 pg/ml). Cultures containing TCC and APC alone served as negative controls. TCC with a ratio of IFN-γ/IL-4>10 were classified as Th1, 0.1-10 as Th0 and <0.1 as Th2.

### Results :

Single Cys-mutants of Art v 1 (C22S, C47S, C49S) and the Defensin-domain of Art v 1 (DefWT) plus its Cys-mutants (DefC22S, DefC26S, DefC47S, DefC49S) were studied for their capacity to stimulate T cells of mugwort pollen-allergic individuals using 1) PBMC, 2) allergen-specific TCL and 3) TCC:

### PBMC

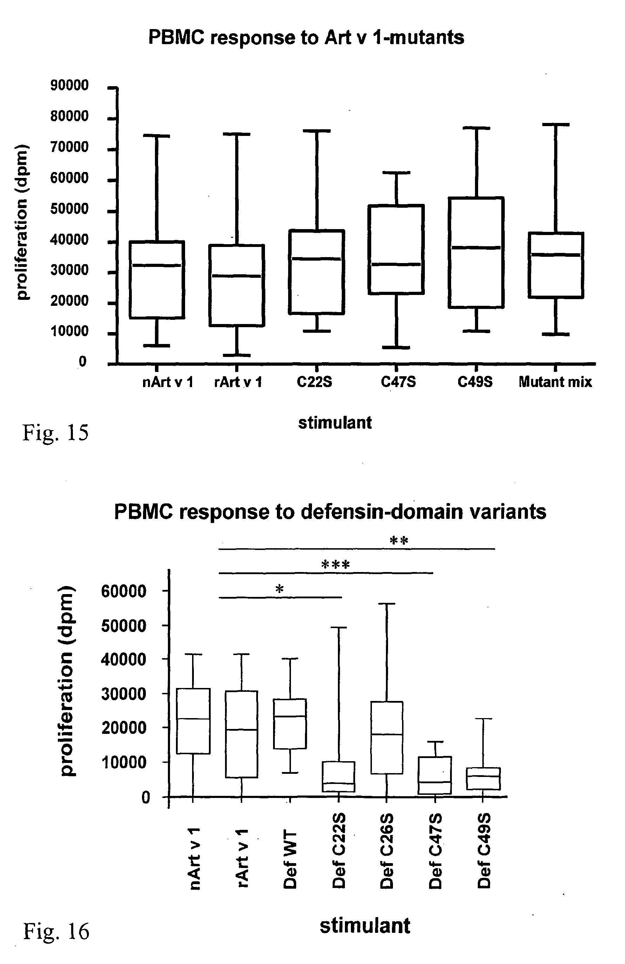

PBMC derived from different mugwort pollen-allergic individuals (n=14), were stimulated with nArt v 1, rArt v 1, C22S, C47S, and C49S or an equimolar mixture of C22S, C47S, and C49S in concentrations varying from 20-0.6 µg/ml. To achieve optimum proliferative responses, lower concentrations of the mutants were needed as compared to rArt v 1. The means of the optimum concentrations were 5.6 for nArt v 1 (p=0.005; Wilcoxon Signed Ranks Test; comparison to rArt v1), 12.1 µg/ml for rArt v 1, 3.1 µg/ml for C22S (p=0.001), 9.6 µg/ml for C47S (not significant), 5.4 µg/ml for C49S (p=0.020) and 4.2 µg/ml (p=0.023) for the mutant-mixture. As compared to Art v 1, the median values obtained with the mutants were also higher, however these differences were not statistically significant (Fig. 15).

The C26-variant of the defensin-domain (DefC26S) was produced and included in the following proliferation assays, because in the meantime the present studies indicated that the cysteine at position 26 is only rarely involved in T cell activation. The defensin-variants of Art v 1 were tested with PBMC from mugwort pollen-allergic donors (n=18). Proliferation induced by DefWT and DefC26S were comparable to rArt v 1 and nArt v 1 whereas DefC22S (p=0.039), DefC47S ( p=0.001) and DefC49S (p=0.003) were significantly weaker stimulants (Fig. 16). The optimum concentrations for PBMC-stimulations were similar for all stimulants tested.

### TCL and TCC

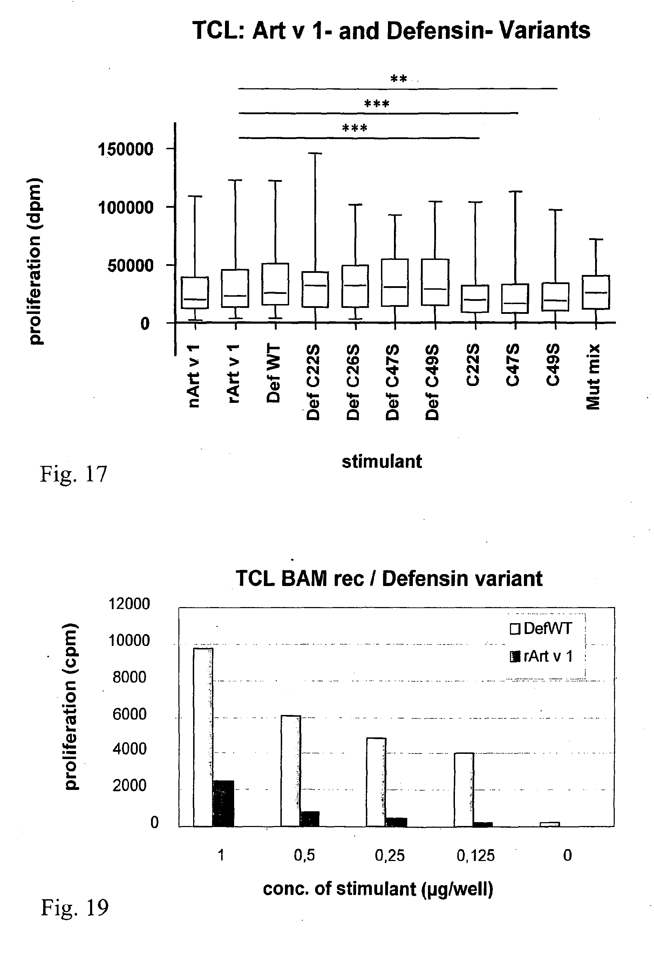

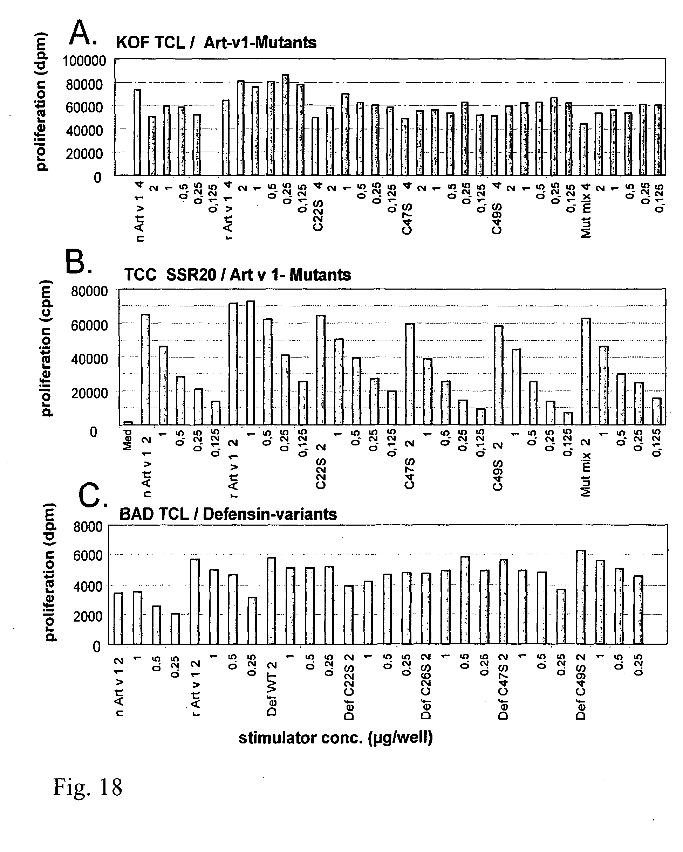

Twenty-two TCL derived from 18 different patients were stimulated with Art v 1 or C22S, C47S, and C49S (n=22) or an equimolar mixture of C22S, C47S, and C49S (n=8/22); Fig. 17). In contrast to PBMC, for stimulation of TCL or TCC, the optimum protein concentration of each protein for stimulation of proliferation was very similar for all variants of Art v 1. A representative example is shown in Fig. 18 A.). The Art v 1 mutants (all p<0.001) and nArt v 1 (p=0.001) induced significantly lower proliferative responses than rArt v 1. Based on a lower number of experiments, the mutant mixture seemed similarly active as rArt v 1.

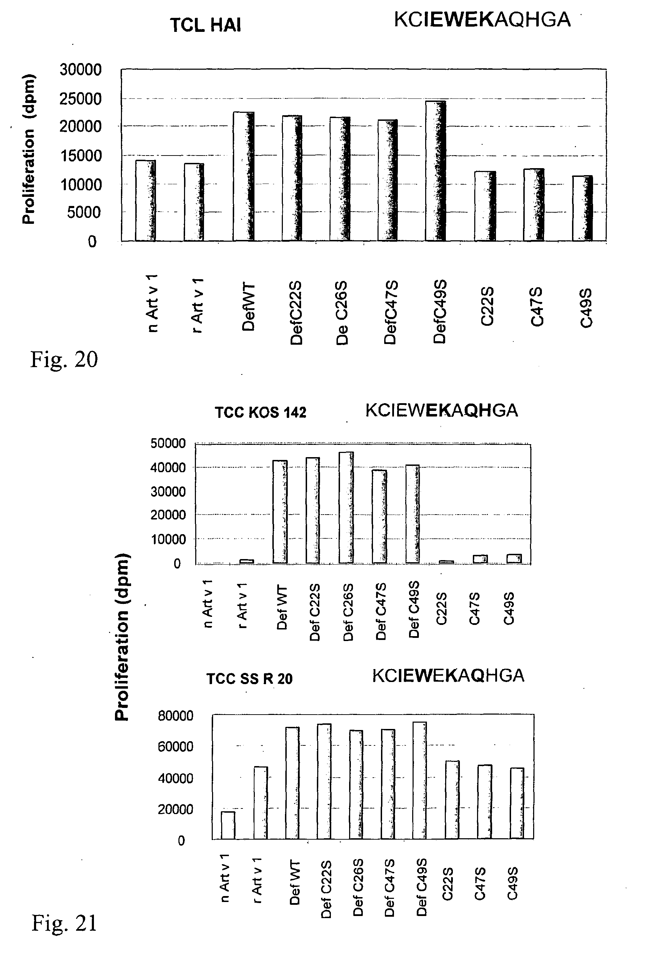

The stimulatory capacity of DefWT, DefC22S, DefC26S, DefC47S and DefC49S was tested in 17 Art v 1-specific TCL derived from 12 different patients. Similar to the observation in PBMC, the Defensin-domain was a better stimulus for Art v 1-specific TCL than complete Art v 1 (Fig. 17; 19) or its mutants (Fig. 20), although the difference did not reach statistical significance. The increased response may be due to a better uptake or processing of the defensin domain by antigen-presenting cells as compared to the complete molecule.

In addition to PBMC and TCL, proliferation of 16 different Art v 1-specific TCC (derived from 6 patients) after stimulation with Art v 1 variants (5µg/ml) was analyzed. The individual reactivity of TCC to each mutant varied according to the core amino acids recognized within Art v 1₂₅₋₃₆, but overall there were no significant differences observed between rArt v 1 and its variants. One example is depicted in Fig. 18B.

Defensin-variants were compared to Art v 1 and its mutants in 6 TCC from 4 different mugwort pollen-allergic donors. In general DefWT (and the Defensin-variants containing the relevant core amino acids) induced higher proliferative and cytokine- responses than Art v 1 or Art v 1 mutants, substantiating the superiority of the defensin-domain regarding the stimulation of Art v 1-specific T lymphocytes. Fig. 21 shows 2 representative experiments.

### Example 3: Immunodominant Peptide of Art v 1 for Immunotherapy

In addition to hypoallergens, small linear synthetic peptides, merely representing HLA class II-restricted T cell epitopes incapable of cross-linking cell-bound IgE, have been considered for tolerance induction in type I allergy. Besides increased safety, immunotherapy with peptides as alternative to allergen-extracts used in conventional SIT would offer the advantages of inexpensive production, as well as ease of purification, standardization and storage.

### 3.1. Peptide design

The previous identification of T cell epitopes within Art v 1 by proliferation assays using 12-mer synthetic peptides resulted in a single immunodominant epitope: Art v 1₂₅₋₃₆ (KCIEWEKAQHGA) (SEQ ID No. 52). Since the N-terminally neighbouring peptides showed additional T cell stimulatory capacity in some patients, not only the 12-mer but also longer peptides containing the immunodominant epitope plus possible additional epitopes were considered as peptide vaccine for SIT. The 12-mer peptide KCIEWEKAQHGA (Art v 1₂₅₋₃₆) (SEQ ID No. 52) was recognized by 85% out of 72 patients; a 15-mer CDKKCIEWEKAQHGA (Art v 1₂₂₋₃₆) (SEQ ID No. 53) would cover 92% of patients and a 18-mer NKKCDKKCIEWEKAQHGA (Art v 1₁₉₋₃₆) (SEQ ID No. 54) would cover 94.1% of patients. Cysteine residues in general are highly prone to form intra-/intermolecular disulfide-bonds and implicate a risk of intramolecular cyclisation or dimerization. Therefore the terminal C was removed from the 15-mer resulting in a 14-mer. Within the 18-mer single or both cysteins were exchanged by alanine or serine. The designed peptides were tested in silico for hydrophobicity, solubility and stability using ProtParam www.expasy.org. Best results were obtained for the following peptide molecules that were chosen for further investigations.
12-mer: KCIEWEKAQHGA (Art v 1₂₅₋₃₆) (SEQ ID No. 52
14-mer: DKKCIEWEKAQHGA (Art v 1₂₃₋₃₆) (SEQ ID No. 55)

| 18-mers: NKKCDKKCIEWEKAQHGA mutant peptides: | (Art v 1₁₉₋₃₆) | and its cysteine |
|---|---|---|
| NKKADKKCIEWEKAQHGA | (AC) | SEQ ID No. 8 |
| NKKSDKKCIEWEKAQHGA | (SC) | SEQ ID No. 9 |
| NKKADKKAIEWEKAQHGA | (AA) | SEQ ID No. 10 |
| NKKADKKSIEWEKAQHGA | (AS) | SEQ ID No. 11 |
| NKKSDKKSIEWEKAQHGA | (SS) | SEQ ID No. 12 |
| NKKSDKKAIEWEKAQHGA | (SA) | SEQ ID No. 13 |

### Biochemical testing

Peptides were purchased as non-modified molecules from Thermo Electron Corporation (Ulm/ Germany). All peptides proved to be instantly soluble in distilled water at a concentration of 2 mg/ml.

The purity and quality of the peptides was verified in mass analysis and Reverse phase-HPLC. All candidate peptides showed the correct mass without any modification and purity of the preparations was demonstrated. The 18-mer (NKKCDKKCIEWEKAQHGA) (SEQ ID No. 54) harbouring two cysteine residues formed a cyclic peptide due to an intramolecular disulfide bond.

### IgE-binding of peptides

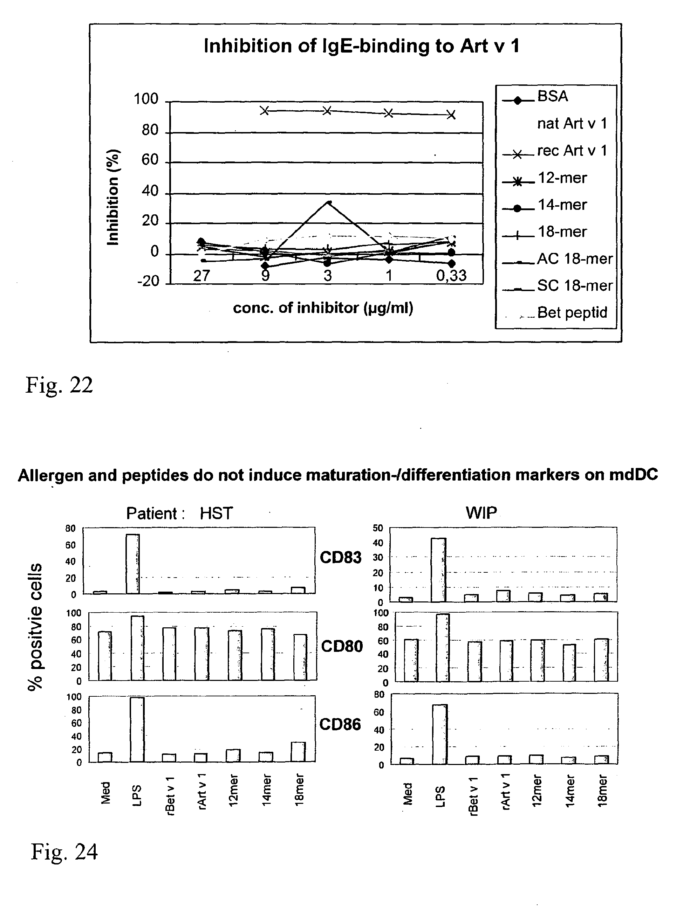

Possible IgE-binding by the peptides was assessed by means of inhibition ELISA. A serum pool derived from 10 mugwort pollen-allergic patients was preincubated with either recombinant Art v 1 or natural Art v 1 or different peptides (12-mer, 14-mer, 18-mer: CC, AC and SC variants) at different concentrations. The binding of this serum pool to Art v 1 was strongly inhibited by rArt v 1 and nArt v 1, but not by any of the peptides (Fig. 22) indicating that none of these peptides exhibits a significant binding of IgE.

### Mediator release

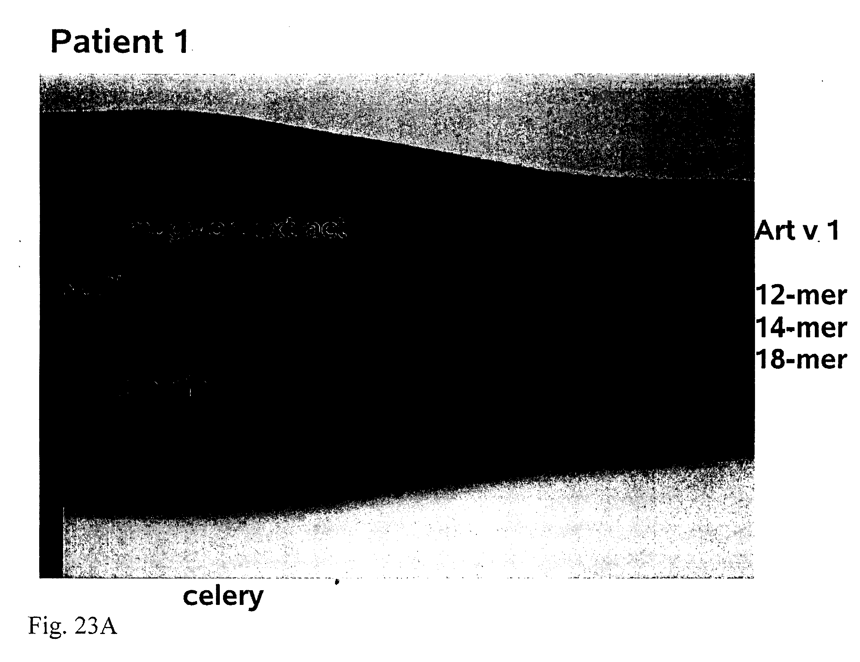

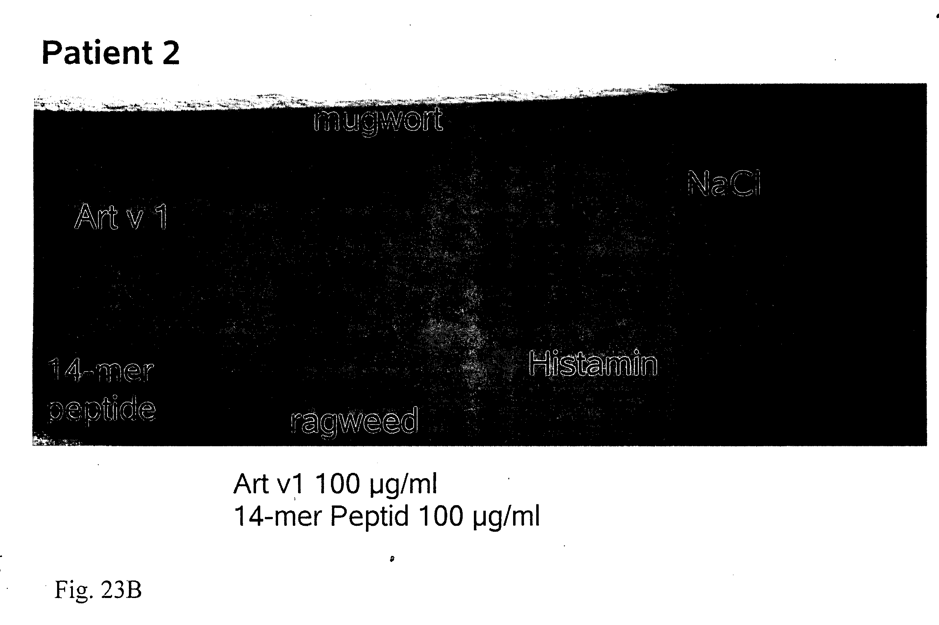

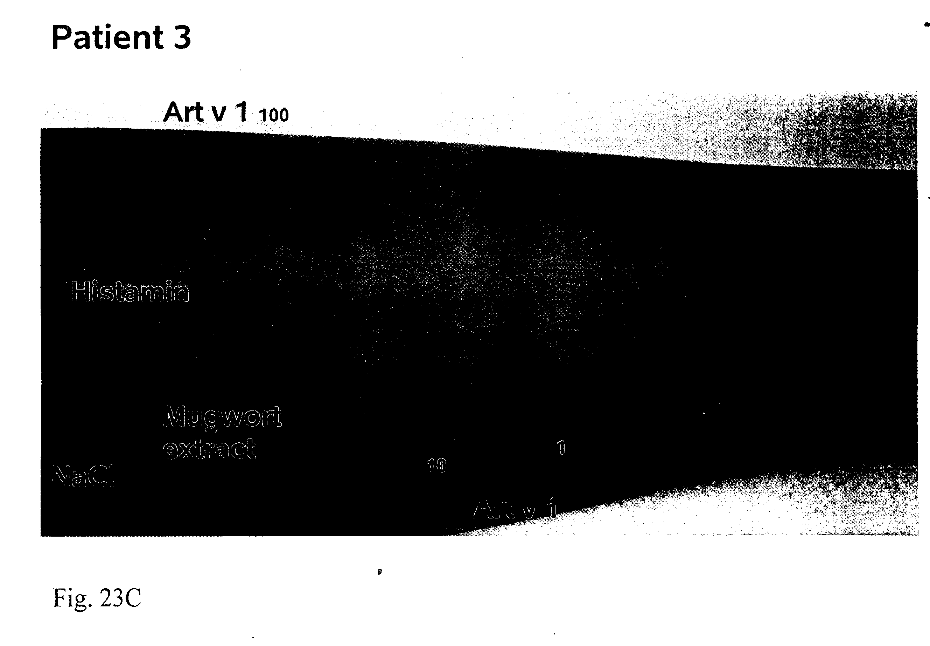

The potency of the Art v 1 peptides to induce mediator release was tested *in vivo*. Skin-Prick-tests were performed in 3 patients with mugwort pollen allergy (Fig 23). In contrast to mugwort pollen extract and rArt v 1 (100 µg/ml is about 9.3 µM), the peptides did not elicit any skin reactions in solutions up to 100 µg/ml(about 90 µM for the 12-mer, 71 µM for the 14-mer and 58 µM for the 18-mer peptide). These results demonstrate the hypoallergenic nature of the peptides and support their use for a peptide-based immunotherapy approach.

### HLA-binding of peptides

HLA-binding of the 12-mer peptide to the most frequent HLA-DR-alleles in Caucasians was tested *in vitro* using a competition assay (ref. for method: B. Maillere et al., Eur J Immunol. 2002; 32:3699-3707). DR1, DR3 and DR4 were efficient, DR13, DRB5 moderate and DR11 and DR15 weak binders of the 12-mer. DR7, DRB3, and DRB4 showed no binding activity. These data indicate that the 12-mer peptide is partly promiscuous in HLA-binding. Therefore, it may be assumed that not only HLA-DR1-expressing patients with mugwort pollen allergy are able to present Art v 1₂₅₋₃₆. Parallel *in vitro* experiments performed with antigen-presenting cells homozygous for HLA-DR4 and Art v 1₂₅₋₃₆-specific TCC substantiated this assumption. Isoleucine at amino acid position 27 in Art v 1 turned out to be the most relevant anchor residue for Art v 1₂₅₋₃₆ in the binding groove of the HLA-DR1 molecule. According binding studies for the 14- and 18-mer peptides are envisioned.

### Tests concerning the inflammatory potential

For use in peptide immunotherapy, uptake and presentation of peptides *per se* should not activate antigen-presenting cells to produce pro-inflammatory cytokines or up-regulate co-stimulatory molecules. A lack of co-stimulation during T cell activation has been shown to induce specific tolerance in T cells and is one mechanism assumed to be operative in peptide immunotherapy. Experiments were performed to test whether the peptides under consideration induce maturation or differentiation of immature monocyte-derived dendritic cells (mdDC). MdDC were generated from monocytes isolated from PBMC by magnetic cell-sorting using anti-CD14 microbeads (purity>95%). CD14+ cells (1x10⁶/ml) were cultured in 24-well plates in RPMI 1640 medium supplemented with human recombinant IL-4 (1000 U/ml) and GM-CSF (50 ng/ml) and 10% heat-inactivated fetal calf serum for 7 days. The resulting DC populations had lost the monocyte marker CD14 and >70 % of the cells expressed CD1a, a marker for immature DC. MdDC from 2 different individuals were left untreated or stimulated with the control allergen rBet v 1 (10 µg/ml), rArt v 1 (10 µg/ml), 12-, 14- and 18-mer (CC) peptides (10 µg/ml) or LPS (100 ng/ml). After 48 hours, the surface marker expression (HLA-DR, CD1a = marker for immature DC, CD14, CD83 = maturation marker, and co-stimuli: CD80, CD86, CD40) was analyzed by flow cytometry.

Stimulation with Art v 1 or peptides induced no signs of activation or maturation on mdDC. In contrast, LPS - a well-known stimulus for DC maturation- increased the percentage of CD83 -, CD80- and CD86-positive cells (Fig. 24) and the mean fluorescence of HLA-DR, CD80 and CD40.

### Proliferation tests

### A. PBMC

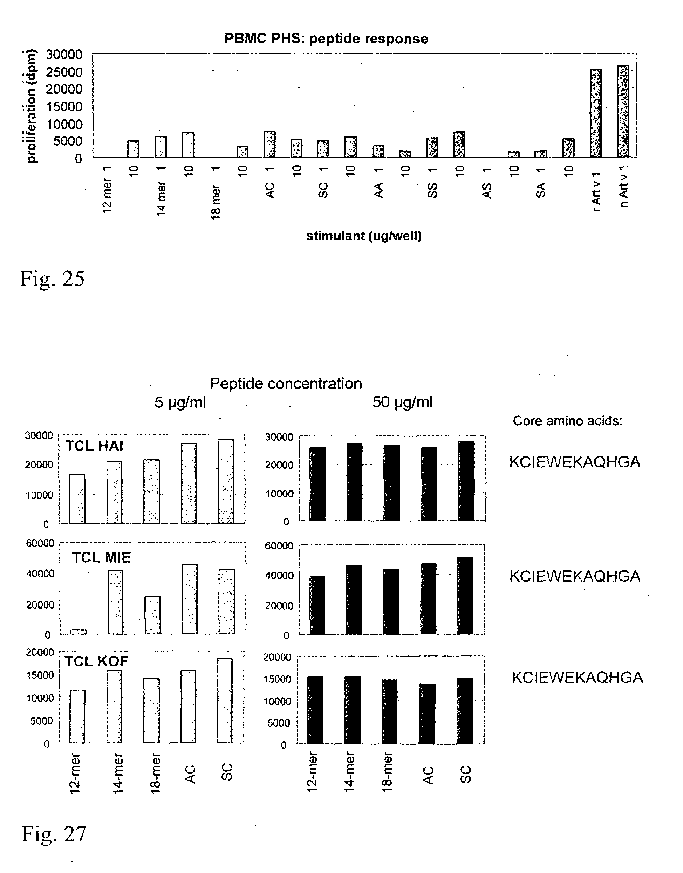

It is known that stimulation of PBMC by peptides containing T cell epitopes result in rather weak proliferations. In the only patient tested so far, the proliferative response of PBMC was best induced with the 14-mer; the 12-mer, the original (CC) 18-mer and AS mutants were inactive at the lower concentration of 5 ug/ml (Fig. 25).

### B. TCL

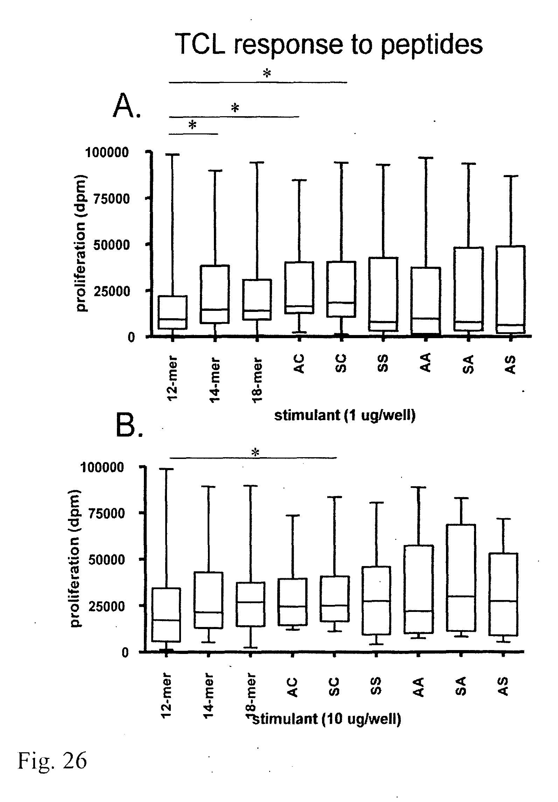

After enrichment of Art v 1-specific T cells from PBMC *in vitro*, 13 TCL Art v 1-specific TCL derived from 9 different mugwort pollen-allergic patients were used for peptide testing. The 14-mer was a significantly better stimulus for proliferation (n=13 TCL; p=0.043; Wilcoxon Signed Ranks test) compared to the 12-mer. Interestingly, the 18-mer (although exhibiting an intramolecular cyclic C-C modification) also induced higher proliferation (not statistically significant) (Fig. 26A).

The 18-mer variants AC and SC were tested in parallel in 12/13 TCL and were also found to induce significantly higher proliferation than the 12-mer, similar to the 14-mer (Fig. 26 A).

The 18-mer variants SS, AA, SA, AS were also tested in 9 of the TCL. However, the stimulatory capacity of these peptides did not significantly differ from the 12-mer (Fig. 26A).

Differences in stimulatory capacity were less marked with higher concentration of peptides (Fig. 26B). Three representative experiments with TCL from different donors are shown in Fig. 27.

### C. TCC

In 6/7 Art v 1-specific TCC from 4 different patients tested, the 14-mer and 18-mer induced higher proliferative T cell responses than the 12-mer. The 14-mer was a better stimulus than the 18-mer in 4/7 TCC :

| **TCC** | **12-mer** | **14-mer** | **18-mer** | **Medium** |
|---|---|---|---|---|
| **BAM 102** | 7034* | 12213 | 10797 | 543 |
| **BAM 54** | 6827 | 11706 | 13475 | 456 |
| **BAM 6** | 22139 | 44099 | 34397 | 223 |
| **BAM 3** | 8734 | 17578 | 13771 | 221 |
| **SSR20** | 9379 | 35002 | 45367 | 422 |
| **KOS 142** | 15952 | 6854 | 12727 | 428 |
| **PHS R** | 24 17771 | 34332 | 2926 | 110 |
| **median** | **9379** | **17578** | **13475** | |

| | | | | |
|---|---|---|---|---|
| *Proliferation (cpm); peptide concentration 5ug/ml; differences are not statistically significant (but: p<0.05 if KOS 142 is excluded) | | | | |

Different from the other 6 TCC, KOS142 reacted best with the 12-mer; the 14- and 18-mer were less stimulatory for KOS142 and detectable only at a higher concentration of the peptide. However, this observation can be explained by the fact that KOS142 recognizes amino acid residues more C-terminal within Art v 1₂₅₋₃₆ as compared to most other Art v 1-reactive T cells characterized so far (e.g. compare core amino acids denoted in Fig. 26/27). It is assumed that the 12-mer peptide binds to HLA-class II molecules in another region, which seems to be less accessible for the 14-mer or 18-mer peptide.

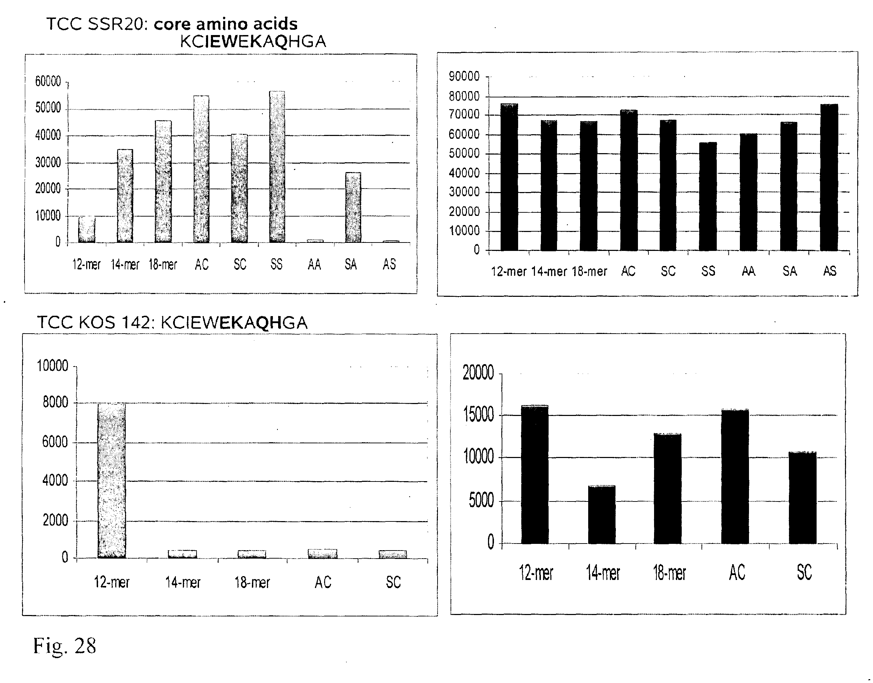

In addition, the other 18-mer peptide variants were also tested in 2 TCC (Fig. 28). For TCC SSR20, the variant SS and SA were better stimulators than the 12-mer peptide, AA and AS induced T cell proliferation only at a higher peptide concentration.

TCC PHSR24 was activated by the 14-mer, but also by the 18-mer variants AC and SC. However, the original 18-mer and variants with double cysteine mutations, i.e. AA, SS, AS and SA did not activate the TCC, not even at the higher concentration 50µg/ml (Fig. 28). These results are in line with C26 being an essential amino acid of the T cell epitope core recognized by this TCC. However, C26 is not crucial for recognition by most other Art v 1₂₅₋₃₆-specific TCC or TCL (Jahn-Schmid et al., J Allergy Clin Immunol 115:399-404, 2005).

### Comparison of stability: peptides subjected to repeated freeze/thaw/37°C:

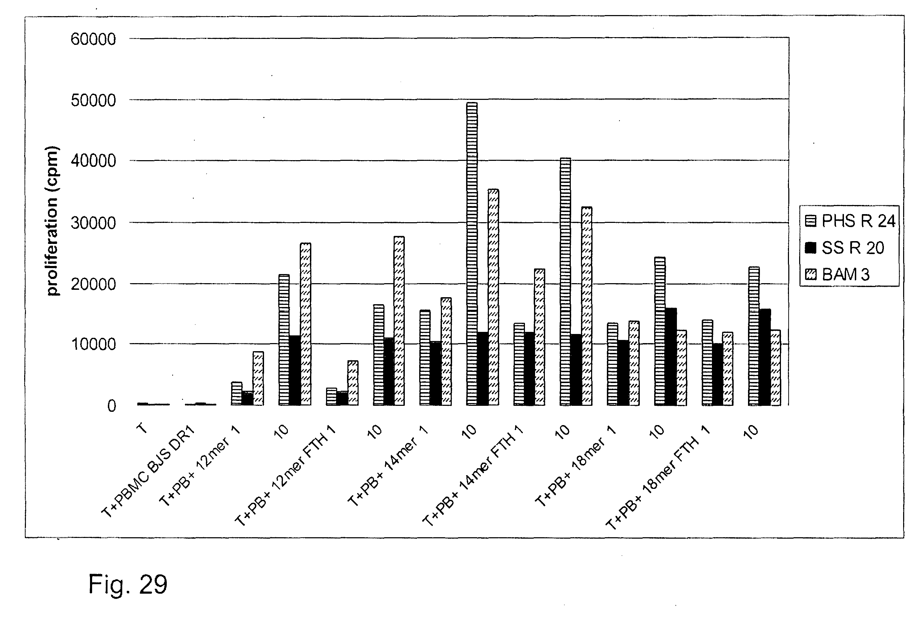

Stock solutions (2 mg/ml distilled water) of all peptides under investigation were repeatedly (3x) frozen at -20°C, thawed and incubated for 1 hour at 37°C hrs. The proliferation induced by freeze-thawed peptides as compared to non-treated peptides was evaluated in 3 different Art v 1-specific TCC (Fig. 29). No difference before and after the freeze-thawing was observed, indicating a reasonable and similar stability for all peptides.

### Summary

In Europe and Asia, the incidence of allergic diseases caused by mugwort pollen is about 14%. Due to climatic changes *Artemisia vulgaris* is constantly spreading and may therefore pose an increased risk for development of mugwort allergy in predisposed individuals. Art v 1 was identified as major mugwort pollen allergen, which is recognized by IgE from 95% of mugwort-allergic patients. At present specific immunotherapy (SIT) is the only curative treatment for type I allergies. In contrast to allergen extracts, which are routinely used for diagnosis and therapy of type I allergy, well-defined molecules/peptides that are easy to standardize and characterize are propose here. To overcome side-effects typically encountered during SIT the major aim was the generation of molecules/peptides with low IgE binding capacity. These hypoallergens or peptides are candidate molecules for treatment of type I allergy in mugwort allergic individuals, designed for a safer and more efficient immunotherapy.

The three-dimensional structure of the N-terminal defensin-like domain of Art v 1 is stabilized by eight cysteines presumably involved in the formation of four disulfide bonds. The formation of these intramolecular cysteine bridges is very important for the allergenicity of the molecule, as upon reduction and alkylation of the protein IgE binding activity is destroyed. Using site-directed mutagenesis, cysteine residues were in turn exchanged for serine and alanine, which represent the most common amino acids for cysteine substitution. All constructs were expressed as histidine tagged fusion proteins in E. *coli* and purified. Immunoblots using sera from Art v 1-allergic patients showed a tremendous decrease in the IgE binding capacity of four variants targeting cysteine residues at position 22, 26, 47, and 49. Art v 1 variants C22S, C47S, and C49S were expressed in E. coli, purified and characterized in detail; mutant C26S was not included as previously was found that this cysteine residue was critical for the stimulation of some Art v 1-specific T cells. ELISA and cross-inhibition experiments performed with recombinant wild-type Art v 1 and variants showed significant reduction in the IgE-binding capacity of the Art v 1 mutants. The allergenic activity of these proteins was determined by degranulation of transfected rat basophil cells. The experiments showed a reduction in the capacity to induce mediator release of at least 50,000 fold compared to wild-type rArt v 1. These results demonstrate the hypoallergenic character of variants C22S, C47S, and C49S, as this *in vitro* experiment resembles the biological activity of the proteins in an allergic reaction. As T cells play an important role for a successful SIT, experiments were performed to compare the potency of the hypoallergenic Art v 1 variants to induce allergen-specific T cell proliferation. All molecules were able to stimulate PBMC as well as most of the Art v1₂₅₋₃₆-specific TCL and TCC, in a similar fashion as wild-type recombinant and natural Art v 1 proteins.

The defensin domain part of Art v 1 was expressed as recombinant protein and was found to exhibit the same immunological properties as full-length Art v 1. Since the production of the globular domain alone is more efficient and all immunological properties are restricted to the N-terminal domain, also mutants of the Art v 1-defensin domain were generated. Partial immunological characterizations were performed with all defensin mutants, and the hypoallergenic properties seem to be the same as those observed for full-length Art v 1 mutants. In addition, double mutants C22S-C47S and C26S-C49S of the defensin domain were generated to prevent intermolecular disulfide bond formation. All molecules that demonstrated a hypoallergenic characteristic (Art v 1 full-length C22S, C47S, C49S and defensin variants C22S, C26S, C47S, and C49S) could be used as candidate molecules for treatment of mugwort type I allergy. Mugwort allergy is clinically and serologically often associated with ragweed allergy. A recent study by Asero *et al*. demonstrated that patients exposed to both mugwort and ragweed pollen are co-sensitized. In other words, allergic symptoms elicited by pollen exposure during autumn season in areas where both plants are growing are not due to cross-reactivity of homologous allergens contained in mugwort and ragweed pollen. Instead, genuine sensitization by the major mugwort and ragweed pollen allergens, Art v 1 and Amb a 1 respectively, takes place. Therefore, it was suggested that SIT for these co-sensitized patients should include allergens from both plant sources. It is proposed to develop fusion constructs containing the relevant T cell epitopes of the major ragweed and mugwort allergens for the treatment of patients sensitized to both weeds. Fusions of Art v 1 variants or defensin variants in combination with the alpha chain of Amb a 1 will be generated and tested for the hypoallergenic capacity as well as the potency to stimulate T cells derived from mugwort and ragweed allergic patients.

Previous T-cell epitope mapping experiments using 12-mer synthetic peptides identified a single immunodominant epitope in the Art v 1 molecule (KCIEWEKAQHGA) (SEQ ID No. 52). Further characterizations of the T cell response using longer peptides to cover the reactivity of more patients were performed with a 14-mer (DKKCIEWEKAQHGA) (SEQ ID No. 55), an 18-mer (NKKCDKKCIEWEKAQHGA) (SEQ ID No. 54) and cysteine analogues of the 18-mer. All peptides used were biochemically characterized and found to be pure, non-modified and stable. Inhibition ELISA experiments demonstrated that all peptides lacked IgE binding activity and would therefore not trigger an allergic reaction mediated by IgE cross-linking. All peptides also failed to induce a typical inflammatory response in antigen presenting dendritic cells in vitro. Regarding the proliferation of Art v 1-specific TCL and TCC, the 14-mer (DKKCIEWEKAQHGA) (SEQ ID No. 55) performed best in most experiments. Besides, the 18-mer and distinct peptide analogues were also able to stimulate specific T-cells but varied between the tested lines and clones. Peptides designed for immunotherapy should be able to stimulate T cells of most allergic individuals, therefore the 14-mer is proposed as candidate molecule, which should cover about 90% of Art v 1-allergic patients. In addition, mixtures of the immunodominant peptide DKKCIEWEKAQHGA (SEQ ID No. 55) of Art v 1 together with 3 or 4 peptides of the major ragweed allergen Amb a 1 (GMIKSNDGPPIL (SEQ ID NO. 56), GSSQIWIDHCSLSKS SEQ ID NO. 57), DKDLLENGAIFVTSGSDPVLTPVQ (SEQ ID NO. 58), AIKLTSSAGVLSCRP (SEQ ID NO. 59)) would provide therapeutic peptides suited for treatment of mugwort and ragweed co-sensitized patients.

## Claims

1. Hypoallergenic variant of the mugwort allergen Art v 1 (SEQ ID No. 1) or a fragment thereof, wherein the variant comprises at least one modification of at least one cysteine residue at amino acid positions 22, 26, 47 or 49 of SEQ ID No. 1.

2. Variant according to claim 1, **characterised in that** the at least one modification comprises an amino acid substitution, an amino acid deletion or a chemical modification in which the sulfur group of the cysteine residue is joined to a carbon atom or sulfur atom of a sulfur blocking group.

3. Variant according to claim 1 or 2, **characterised in that** the at least one cysteine residue is substituted by at least one amino acid residue selected from the group consisting of serine, alanine, or glycine.

4. Variant according to any one of claims 1 to 3, **characterised in that** the variant comprises at least one further modification of at least one further cysteine residue at amino acid positions 6, 17, 37 or 53 of SEQ ID No. 1.

5. Variant according to any one of claims 1 to 4, **characterised in that** the fragment comprises amino acid residues 1 to 55 of SEQ ID No. 1, preferably amino acid residues 19 to 36 of SEQ ID No. 1, more preferably amino acid residues 23 to 36 of SEQ ID No. 1, in particular amino acid residues 25 to 36 of SEQ ID No. 1.

6. Variant according to any one of claims 1 to 5, **characterised in that** the variant comprises an amino acid sequence selected from the group consisting of SEQ ID No. 2 to 13.

7. Nucleic acid molecule encoding a hypoallergenic variant according to any one of claims 1 to 6.

8. Vector comprising a nucleic acid molecule according to claim 7.

9. Host cell comprising a nucleic acid molecule according to claim 7 or a vector according to claim 8.

10. Pharmaceutical preparation comprising at least one hypoallergenic variant according to any one of claims 1 to 6, a nucleic acid molecule according to claim 7 or a vector according to claim 8.

11. Use of at least one hypoallergenic variant according to any one of claims 1 to 6, a nucleic acid molecule according to claim 7 or a vector according to claim 8 for the manufacture of a pharmaceutical preparation, preferably vaccine, for preventing or treating a mugwort pollen allergy in an individual, in particular an allergy caused by Art v 1, or an allergy cross-reacting with a mugwort pollen allergy.


SEQUENCE LISTING

<110>  Biomay AG

<120>  Hypoallergenic variants

<130>  R 47543

<160>  67

<170>  PatentIn version 3.4

<210>  1
<211>  108
<212>  PRT
<213>  Artemisia vulgaris

<400>  1

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  2
<211>  108
<212>  PRT
<213>  Artificial

<220>
<223>  Art v 1 variant

<400>  2

Ala Gly Ser Lys Leu Cys Glu Lys Thr Ser Lys Thr Tyr Ser Gly Lys
1               5                   10                  15

Cys Asp Asn Lys Lys Ser Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro

50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  3
<211>  108
<212>  PRT
<213>  Artificial

<220>
<223>  Art v 1 variant

<400>  3

Ala Gly Ser Lys Leu Cys Glu Lys Thr Ser Lys Thr Tyr Ser Gly Lys
1               5                   10                  15

Cys Asp Asn Lys Lys Cys Asp Lys Lys Ser Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  4
<211>  108
<212>  PRT
<213>  Artificial

<220>
<223>  Art v 1 variant

<400>  4

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Ser Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210> 5
<211> 108
<212> PRT
<213> Artificial

<220>
<223> Art v 1 variant

<400> 5

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Ser Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210> 6
<211> 108
<212> PRT
<213> Artificial

<220>
<223> Art v 1 variant

<400> 6

Ala Gly Ser Lys Leu Cys Glu Lys Thr Ser Lys Thr Tyr Ser Gly Lys

1                   5                   10                  15

Cys Asp Asn Lys Lys Ala Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  7
<211>  108
<212>  PRT
<213>  Artificial

<220>
<223>  Art v 1 variant

<400>  7

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Ala Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  8
<211>  18
<212>  PRT
<213>  Artificial

<220>
<223>   Art v 1 fragment variant

<400>   8

Asn Lys Lys Ala Asp Lys Lys Cys Ile Glu Trp Glu Lys Ala Gln His
1               5                   10                  15

Gly Ala

<210>   9
<211>   18
<212>   PRT
<213>   Artificial

<220>
<223>   Art v 1 fragment variant

<400>   9

Asn Lys Lys Ser Asp Lys Lys Cys Ile Glu Trp Glu Lys Ala Gln His
1               5                   10                  15

Gly Ala

<210>   10
<211>   18
<212>   PRT
<213>   Artificial

<220>
<223>   Art v 1 fragment variant

<400>   10

Asn Lys Lys Ala Asp Lys Lys Ala Ile Glu Trp Glu Lys Ala Gln His
1               5                   10                  15

Gly Ala

<210>   11
<211>   18
<212>   PRT
<213>   Artificial

<220>
<223>   Art v 1 fragment variant

<400>   11

Asn Lys Lys Ala Asp Lys Lys Ser Ile Glu Trp Glu Lys Ala Gln His
1               5                   10                  15

Gly Ala

<210>   12
<211>   18
<212>   PRT

<213>  Artificial

<220>
<223>  Art v 1 fragment variant

<400>  12

Asn Lys Lys Ser Asp Lys Lys Ser Ile Glu Trp Glu Lys Ala Gln His
1               5                   10                  15

Gly Ala

<210>  13
<211>  18
<212>  PRT
<213>  Artificial

<220>
<223>  Art v 1 fragment variant

<400>  13

Asn Lys Lys Ser Asp Lys Lys Ala Ile Glu Trp Glu Lys Ala Gln His
1               5                   10                  15

Gly Ala

<210>  14
<211>  36
<212>  DNA
<213>  Artificial

<220>
<223>  Primer

<400>  14
gctggttcaa agttgtctga aaagacaagc aagacg                                36

<210>  15
<211>  36
<212>  DNA
<213>  Artificial

<220>
<223>  Primer

<400>  15
gctggttcaa agttggctga aaagacaagc aagacg                                36

<210>  16
<211>  37
<212>  DNA
<213>  Artificial

<220>
<223>  Primer

<400>  16
gacgtattcg ggtaagtccg acaacaagaa atgtgac                               37

<210>  17

<211>  37
<212>  DNA
<213>  Artificial

<220>
<223>  Primer

<400>  17
gacgtattcg ggtaaggccg acaacaagaa atgtgac                                37


<210>  18
<211>  48
<212>  DNA
<213>  Artificial

<220>
<223>  Primer

<400>  18
cgggtaagtg cgacaacaag aaatctgaca aaaagtgtat agagtggg                    48


<210>  19
<211>  48
<212>  DNA
<213>  Artificial

<220>
<223>  Primer

<400>  19
cgggtaagtg cgacaacaag aaagctgaca aaaagtgtat agagtggg                    48


<210>  20
<211>  33
<212>  DNA
<213>  Artificial

<220>
<223>  Primer

<400>  20
gtgacaaaaa gtctatagag tgggagaaag cgc                                    33


<210>  21
<211>  33
<212>  DNA
<213>  Artificial

<220>
<223>  Primer

<400>  21
gtgacaaaaa ggctatagag tgggagaaag cgc                                    33


<210>  22
<211>  33
<212>  DNA
<213>  Artificial

<220>
<223>  Primer

<400>  22
caacatggtg cttctcacaa gagagaagcc ggc                                    33

<210>  23
<211>  38
<212>  DNA
<213>  Artificial

<220>
<223>  Primer

<400>  23
gccggcaaag aaagttcctt ttgctacttt gactgtcc                                38


<210>  24
<211>  39
<212>  DNA
<213>  Artificial

<220>
<223>  Primer

<400>  24
gccggcaaag aaagttgctt ttcctacttt gactgttcc                               39


<210>  25
<211>  39
<212>  DNA
<213>  Artificial

<220>
<223>  Primer

<400>  25
gccggcaaag aaagttgctt tgcctacttt gactgttcc                               39


<210>  26
<211>  37
<212>  DNA
<213>  Artificial

<220>
<223>  Primer

<400>  26
gcttttgcta ctttgactct tccaaatcgc ctcctgg                                 37


<210>  27
<211>  37
<212>  DNA
<213>  Artificial

<220>
<223>  Primer

<400>  27
gcttttgcta ctttgacgct tccaaatcgc ctcctgg                                 37


<210>  28
<211>  36
<212>  DNA
<213>  Artificial

<220>
<223>  Primer

<400>  28

cgtcttgctt gtcttttcag acaactttga accagc                                36

<210>  29
<211>  36
<212>  DNA
<213>  Artificial

<220>
<223>  Primer

<400>  29
cgtcttgctt gtcttttcag ccaactttga accagc                                36

<210>  30
<211>  35
<212>  DNA
<213>  Artificial

<220>
<223>  Primer

<400>  30
cacatttctt gttgtcggac ttacccgaat acgtc                                 35

<210>  31
<211>  35
<212>  DNA
<213>  Artificial

<220>
<223>  Primer

<400>  31
cacatttctt gttgtcggcc ttacccgaat acgtc                                 35

<210>  32
<211>  47
<212>  DNA
<213>  Artificial

<220>
<223>  Primer

<400>  32
cccactctat acactttttg tcagatttct tgttgtcgca cttaccc                     47

<210>  33
<211>  47
<212>  DNA
<213>  Artificial

<220>
<223>  Primer

<400>  33
cccactctat acactttttg tcagctttct tgttgtcgca cttaccc                     47

<210>  34
<211>  38
<212>  DNA
<213>  Artificial

<220>
<223>  Primer

<400>  34
gcgctttctc ccactctata gactttttgt cacatttc                                38

<210>  35
<211>  38
<212>  DNA
<213>  Artificial

<220>
<223>  Primer

<400>  35
gcgctttctc ccactctata gcctttttgt cacatttc                                38

<210>  36
<211>  36
<212>  DNA
<213>  Artificial

<220>
<223>  Primer

<400>  36
gccggcttct ctcttgtgag aagcaccatg ttgcgc                                  36

<210>  37
<211>  39
<212>  DNA
<213>  Artificial

<220>
<223>  Primer

<400>  37
ggaacagtca aagtagcaaa aggaactttc tttgccggc                               39

<210>  38
<211>  44
<212>  DNA
<213>  Artificial

<220>
<223>  Primer

<400>  38
ggcgatttgg aacagtcaaa gtaggaaaag caactttctt tgcc                         44

<210>  39
<211>  44
<212>  DNA
<213>  Artificial

<220>
<223>  Primer

<400>  39
ggcgatttgg aacagtcaaa gtaggcaaag caactttctt tgcc                         44

<210>  40
<211>  37
<212>  DNA
<213>  Artificial

<220>
<223>  Primer

<400>  40
ggaggcgatt tggaagagtc aaagtagcaa aagcaac                                37


<210>  41
<211>  37
<212>  DNA
<213>  Artificial

<220>
<223>  Primer

<400>  41
ggaggcgatt tggaagcgtc aaagtagcaa aagcaac                                37


<210>  42
<211>  26
<212>  DNA
<213>  Artificial

<220>
<223>  Primer

<400>  42
gagagaccat ggctggttca aagttg                                            26


<210>  43
<211>  32
<212>  DNA
<213>  Artificial

<220>
<223>  Primer

<400>  43
gagagagact cgagttagtg agtggacgga gg                                     32


<210>  44
<211>  26
<212>  DNA
<213>  Artificial

<220>
<223>  Primer

<400>  44
gagagaccat ggctggttca aagttg                                            26


<210>  45
<211>  36
<212>  DNA
<213>  Artificial

<220>
<223>  Primer

<400>  45
gagagactcg agttacgatt tggaacagtc aaagta                                 36


<210>  46
<211>  33
<212>  DNA

<213>  Artificial

<220>
<223>  Primer

<400>  46
gagagaccat ggcctcctgg agcaacacca gcg                                    33


<210>  47
<211>  33
<212>  DNA
<213>  Artificial

<220>
<223>  Primer

<400>  47
gagagactcg agttagtgag tggacggagg agg                                    33


<210>  48
<211>  56
<212>  PRT
<213>  Artificial

<220>
<223>  Art v 1 fragment

<400>  48

Ala Gly Ser Lys Leu Cys Glu Lys Thr Ser Lys Thr Tyr Ser Gly Lys
1               5                   10                  15


Cys Asp Asn Lys Lys Cys Asp Lys Lys Cys Ile Glu Trp Glu Lys Ala
            20                  25                  30


Gln His Gly Ala Cys His Lys Arg Glu Ala Gly Lys Glu Ser Cys Phe
        35                  40                  45


Cys Tyr Phe Asp Cys Ser Lys Ser
    50                  55


<210>  49
<211>  53
<212>  PRT
<213>  Artificial

<220>
<223>  Art v 1 fragment

<400>  49

Pro Pro Gly Ala Thr Pro Ala Pro Pro Gly Ala Ala Pro Pro Pro Ala
1               5                   10                  15


Ala Gly Gly Ser Pro Ser Pro Pro Ala Asp Gly Gly Ser Pro Pro Pro
            20                  25                  30


Pro Ala Asp Gly Gly Ser Pro Pro Val Asp Gly Gly Ser Pro Pro Pro
        35                  40                  45


Pro Ser Thr His Gly

    50

<210> 50
<211> 46
<212> DNA
<213> Artificial

<220>
<223> Primer

<400> 50
gagactcgag ttacgatttg gaacagtcaa agtagcaaag gaactt 46

<210> 51
<211> 41
<212> DNA
<213> Artificial

<220>
<223> Primer

<400> 51
gagactcgag ttacgatttg gaacagtcaa agtaggaaaa g 41

<210> 52
<211> 12
<212> PRT
<213> Artificial

<220>
<223> Art v 1 fragment

<400> 52

Lys Cys Ile Glu Trp Glu Lys Ala Gln His Gly Ala
1               5                   10

<210> 53
<211> 15
<212> PRT
<213> Artificial

<220>
<223> Art v 1 fragment

<400> 53

Cys Asp Lys Lys Cys Ile Glu Trp Glu Lys Ala Gln His Gly Ala
1               5                   10                  15

<210> 54
<211> 18
<212> PRT
<213> Artificial

<220>
<223> Art v 1 fragment

<400> 54

Asn Lys Lys Cys Asp Lys Lys Cys Ile Glu Trp Glu Lys Ala Gln His
1               5                   10                  15

Gly Ala

<210>  55
<211>  14
<212>  PRT
<213>  Artificial

<220>
<223>  Art v 1 fragment

<400>  55

Asp Lys Lys Cys Ile Glu Trp Glu Lys Ala Gln His Gly Ala
1               5                   10


<210>  56
<211>  12
<212>  PRT
<213>  Artificial

<220>
<223>  Art v 1 fragment

<400>  56

Gly Met Ile Lys Ser Asn Asp Gly Pro Pro Ile Leu
1               5                   10


<210>  57
<211>  15
<212>  PRT
<213>  Artificial

<220>
<223>  Art v 1 fragment

<400>  57

Gly Ser Ser Gln Ile Trp Ile Asp His Cys Ser Leu Ser Lys Ser
1               5                   10                  15


<210>  58
<211>  24
<212>  PRT
<213>  Artificial

<220>
<223>  Art v 1 fragment

<400>  58

Asp Lys Asp Leu Leu Glu Asn Gly Ala Ile Phe Val Thr Ser Gly Ser
1               5                   10                  15

Asp Pro Val Leu Thr Pro Val Gln
            20


<210>  59
<211>  15
<212>  PRT
<213>  Artificial

<220>
<223>  Art v 1 fragment

<400>  59

Ala Ile Lys Leu Thr Ser Ser Ala Gly Val Leu Ser Cys Arg Pro
1               5                   10                  15


<210>  60
<211>  108
<212>  PRT
<213>  Artificial

<220>
<223>  Art v 1 variant

<400>  60

Ala Gly Ser Lys Leu Ser Glu Lys Thr Ser Lys Thr Tyr Ser Gly Lys
1               5                   10                  15


Cys Asp Asn Lys Lys Cys Asp Lys Lys Cys Ile Glu Trp Glu Lys Ala
            20                  25                  30


Gln His Gly Ala Cys His Lys Arg Glu Ala Gly Lys Glu Ser Cys Phe
        35                  40                  45


Cys Tyr Phe Asp Cys Ser Lys Ser Pro Pro Gly Ala Thr Pro Ala Pro
    50                  55                  60


Pro Gly Ala Ala Pro Pro Pro Ala Ala Gly Gly Ser Pro Ser Pro Pro
65                  70                  75                  80


Ala Asp Gly Gly Ser Pro Pro Pro Pro Ala Asp Gly Gly Ser Pro Pro
                85                  90                  95


Val Asp Gly Gly Ser Pro Pro Pro Pro Ser Thr His
            100                 105


<210>  61
<211>  108
<212>  PRT
<213>  Artificial

<220>
<223>  Art v 1 variant

<400>  61

Ala Gly Ser Lys Leu Cys Glu Lys Thr Ser Lys Thr Tyr Ser Gly Lys
1               5                   10                  15


Ser Asp Asn Lys Lys Cys Asp Lys Lys Cys Ile Glu Trp Glu Lys Ala
            20                  25                  30


Gln His Gly Ala Cys His Lys Arg Glu Ala Gly Lys Glu Ser Cys Phe
        35                  40                  45


Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  62
<211>  108
<212>  PRT
<213>  Artificial

<220>
<223>  Art v 1 variant

<400>  62

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Ser His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  63
<211>  108
<212>  PRT
<213>  Artificial

<220>
<223>  Art v 1 variant

<400>  63

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe

35                  40                  45

Cys Tyr Phe Asp Ser Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  64
<211>  108
<212>  PRT
<213>  Artificial

<220>
<223>  Art v 1 variant

<400>  64

Ala Gly Ser Lys Leu Ala Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  65
<211>  108
<212>  PRT
<213>  Artificial

<220>
<223>  Art v 1 variant

<400>  65

Ala Gly Ser Lys Leu Cys Glu Lys Thr Ser Lys Thr Tyr Ser Gly Lys
1               5                   10                  15

Ala Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  66
<211>  108
<212>  PRT
<213>  Artificial

<220>
<223>  Art v 1 variant

<400>  66

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Ala His Lys Arg Glu Ala Gly Lys Glu Ser Cys Phe
        35                  40                  45

Cys Tyr Phe Asp Cys Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105

<210>  67
<211>  108
<212>  PRT
<213>  Artificial

<220>
<223>  Art v 1 variant

<400>  67

Ala Gly Ser Lys Leu Cys Glu Lys Thr Ser Lys Thr Tyr Ser Gly Lys
1               5                   10                  15

Cys Asp Asn Lys Lys Cys Asp Lys Lys Cys Ile Glu Trp Glu Lys Ala
            20                  25                  30

Gln His Gly Ala Cys His Lys Arg Glu Ala Gly Lys Glu Ser Cys Phe
        35                  40                  45

Cys Tyr Phe Asp Ala Ser Lys Ser Pro Pro Gly Ala Thr Pro Ala Pro
    50                  55                  60

Pro Gly Ala Ala Pro Pro Pro Ala Ala Gly Gly Ser Pro Ser Pro Pro
65                  70                  75                  80

Ala Asp Gly Gly Ser Pro Pro Pro Pro Ala Asp Gly Gly Ser Pro Pro
                85                  90                  95

Val Asp Gly Gly Ser Pro Pro Pro Pro Ser Thr His
            100                 105